# EUROPEAN PATENT SPECIFICATION

(11) **EP 3 871 672 B1**
(45) Date of publication and mention of the grant of the patent: **17.08.2022**
(21) Application number: 21153980.4
(22) Date of filing: 28.01.2021
(51) Int. Cl.: A61K 31/366, C07D 311/78, A61K 47/32, A61K 47/10, A61K 36/30, A61K 9/127, A61K 9/06, A61K 45/06, A61K 36/00, A61K 47/46, A61K 9/00, A61P 19/00, A61P 19/08, A61P 19/02, A61P 29/00, A61K 31/216, A61K 31/192, A61K 31/235

(54) **COMFREYNS, ARYLNAPHTHALENE LIGNANS THAT INHIBIT PRO-INFLAMMATORY GENE EXPRESSION, AND PHARMACEUTICAL COMPOSITION COMPRISING THEM**
BEINWELL, ARYLNAPHTHALENLIGNANE ZUR HEMMUNG DER PROINFLAMMATORISCHEN GENEXPRESSION UND PHARMAZEUTISCHE ZUSAMMENSETZUNG DAMIT
CONSOUDES, LIGNANES ARYLNAPHTHALÈNES QUI INHIBENT L'EXPRESSION DE GÈNES PRO-INFLAMMATOIRES ET COMPOSITION PHARMACEUTIQUE LES COMPRENANT

(30) Priority: 27.02.2020 EP 20159707
(43) Date of publication of application: 01.09.2021
(73) Proprietor: The Procter & Gamble Company, Cincinnati, OH 45202 (US)
(72) Inventor: PLAZA, Dr, Alberto, 64289 Darmstadt (DE); PIACENTE, Prof, Sonia, 84084 Fisciano (IT); MASULLO, Prof, Milena, 84084 Fisciano (IT); D'URSO, Dr, Gilda, 84084 Fisciano (IT)
(74) Representative: P&G Patent Germany

(56) References cited:
- WO-A1-2013/167743
- PREDEL H G ET AL: "Efficacy of a Comfrey root extract ointment in comparison to a Diclo-fenac gel in the treatment of ankle distortions: Results of an observer-blind, randomized, multicenter study", PHYTOMEDICINE, ELSEVIER, AMSTERDAM, NL, vol. 12, no. 10, 15 November 2005 (2005-11-15), pages 707-714, XP025323623, ISSN: 0944-7113, DOI: 10.1016/J.PHYMED.2005.06.001 [retrieved on 2005-11-15]
- ADAMS M ET AL: "Medicinal herbs for the treatment of rheumatic disorders-A survey of European herbals from the 16th and 17th century", JOURNAL OF ETHNOPHARMACOLOGY, ELSEVIER IRELAND LTD, IE, vol. 121, no. 3, 30 January 2009 (2009-01-30), pages 343-359, XP025768153, ISSN: 0378-8741, DOI: 10.1016/J.JEP.2008.11.010 [retrieved on 2008-11-18]
- PATWARDHAN S K ET AL: "Coping with arthritis using safer herbal options", INTERNATIONAL JOURNAL OF PHARMACY AND PHARMACEUTICAL SCIENCES, MADHYA PRADESH, IN, vol. 2, no. 1, 1 January 2010 (2010-01-01) , pages 1-11, XP009194479, ISSN: 0975-1491
- GILDA D'URSO ET AL: "LC-ESI-FT-MSn Metabolite Profiling of Symphytum officinale L. Roots Leads to Isolation of Comfreyn A, an Unusual Arylnaphthalene Lignan", INTERNATIONAL JOURNAL OF MOLECULAR SCIENCES, vol. 21, no. 13, 30 June 2020 (2020-06-30) , page 4671, XP055723145, DOI: 10.3390/ijms21134671

## Description

### FIELD OF THE INVENTION

The present invention relates to one or more arylnaphthalene lignan(s), herein called comfreyns or comfreyn compounds, and a pharmaceutic composition comprising them. Example embodiments of the disclosed compounds can be isolated from comfrey *(Symphytum officinale* L.) roots. Certain embodiments of the comfreyn compounds and the pharmaceutic compositions comprising them reduce inflammation by interfering with pro-inflammatory gene expression.

### BACKGROUND OF THE INVENTION

Consumers are looking for effective and natural ways of relieving pain and inhibiting inflammation. *Symphytum officinale* L. (Boraginaceae) known as comfrey, is a perennial herbaceous plant commonly found in Europe, Asia and North America (C. Staiger, Phytother Res 26, 1441-1448, 2012; A. Trifan et al., Food Chem Toxicol 112, 178-187, 2018). Comfrey has been used in folk medicine for the treatment of diarrhea, bronchitis, tuberculosis, ulcers and hemorrhoids. Nowadays, clinical trials have demonstrated the efficacy and safety of its topical preparations. Like other species belonging to the Boraginaceae family, comfrey contains hepatotoxic and carcinogenic pyrrolizidine alkaloids (PAs). Therefore, PA-depleted extracts are used in over-the-counter topical medicines to reduce inflammation and for the treatment of broken bones, tendon damages, painful joints, ankle distortions and muscles (I. Sowa et al., Nat Prod Res 32, 605-609, 2018, M. Adams et al., J. Ethnopharmacol. 121 (2009) 343-359). Indeed, a liquid hydroalcoholic extract of comfrey root was clinically proven to be effective for the treatment of acute upper and lower back pain, arthritis, gonarthrosis, and blunt injuries (B. Grube et al., Phytomedicine 14, 2-10, 2007; R. Koll et al., Phytomedicine 11, 470-477, 2004; H.G. Predel et al., Phytomedicine 12, 707-714, 2005, S.K. Patwardhan et al. Int. J. Pharm. Pharm. Sci., vol 21, no. 13, 2020, p.4671).

It has been reported that the main constituents of comfrey root include allantoin, polyphenols such as rosmarinic acid, lithospermic acid, ellagic acid and caffeic acid, and abundant mucilage and polysaccharides composed of fructose and glucose units (B. Grabias and L. Swiatek, Pharm. Pharmacol. Lett 8, 81-83, 1998; I. Sowa et al., Nat Prod Res 32, 605-609, 2018; C. Staiger Phytother Res 26, 1441-1448, 2012; A. Trifan et al., Food Chem Toxicol 112, 178-187, 2018). Moreover, comfrey roots have yielded a number of oleanolic and bidesmosidic triterpene saponins (V.U. Ahmad et al., Phytochemistry 32, 1003-1006, 1993; F.V. Mohammad et al., Phytochemistry 40, 213-218, 1995). Recently, the phenolic profile of a hydroalcoholic extract from the roots of *Symphytum officinale* L. was reported to contain salvianolic acid isomers (A. Trifan et al., Food Chem Toxicol 112, 178-187, 2018). Comfrey roots also contain hepatoxic pyrrolizidine alkaloids (PAs) with 1,2 unsaturated necine structures including lycopsamine, intermedine, their acetyl and N-oxide derivatives together with the diester symphytine (R.A. Coulombe, Adv. Food Nutr. Res. 45, 61-99, 2003; F. Liu et al., Talanta 80, 916-923, 2009; A. Trifan et al., Food Chem Toxicol 112, 178-187, 2018).

At present, medicinal products from comfrey root commercialized on the European market contain only extracts from pyrrolizidine-depleted plant material (WO2017/068175A1; WO2018/224518A1) or obtained from special cultivars that do not synthesize pyrrolizidine alkaloids (A. Trifan et al., Food Chem Toxicol 112, 178-187, 2018).

Although comfrey's anti-inflammatory and analgesic properties have been proven in clinical studies, the bioactive compounds associated with these therapeutic activities have yet to be identified. Thus, it is the object of the present invention to provide a better understanding of the chemical composition of comfrey roots and to get preliminary insights on the contribution of its specialized metabolites to the anti-inflammatory activity.

### SUMMARY OF THE INVENTION

The present invention relates to one or more arylnaphthalene lignan(s)that have the general structure as shown below in Formula I.

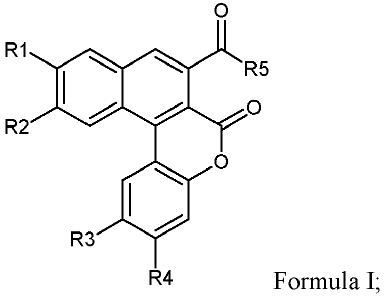

Wherein each R1, R2, R3, and R4 are independently selected from hydrogen, hydroxyl, methyl, methoxy, ethoxy, propoxy, butoxy or pentoxy; and wherein R5 is selected from hydroxyl, methoxy, ethoxy, propoxy, butoxy, pentoxy, feruloyl, caffeoyl, coumaroyl, prenoxy or isoprenoxy residues. In particular, the present invention relates to an arylnaphthalene lignan, named comfreyn A, wherein R1, R2, R3 and R4 each are hydroxyl and R5 is ethoxy as shown in Formula II

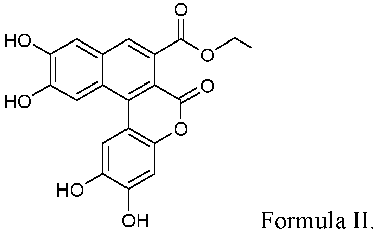

The present invention further relates to a pharmaceutic composition comprising at least one of the one or more arylnaphthalene lignan(s) as disclosed herein in a safe and effective amount and a pharmaceutically acceptable carrier. In particular, the pharmaceutically acceptable carrier may be a carrier for topical application. In addition, or alternatively, the pharmaceutically acceptable carrier may be a carrier for internal administration.

The present invention further relates to the one or more arylnaphthalene lignan(s)as disclosed herein and a pharmaceutic composition comprising them in a safe and effective amount for reduction and/or treatment of inflammation, preferably for the treatment of broken bones, tendon damages, painful joints and muscles, acute upper and lower back pain, gonarthrosis, blunt injuries, for reduction of swelling of joint distortions, for improvement of joint mobility in osteoarthritic joints and/or combinations thereof. In particular, reduction and/or treatment of inflammation is achieved by interfering (blocking or reducing) with pro-inflammatory gene expression.

The present invention further relates to a pharmaceutic composition comprising one or more arylnaphthalene lignan(s) termed comfreyns, and in particular comfreyn A, as active agent in a pharmaceutic carrier, wherein the pharmaceutical formulation may further include a penetration enhancer, or any other functional or non-functional excipient. In a preferred embodiment the pharmaceutic composition comprises comfreyn A in an amount from 3µg/g composition to 5µg/g composition and as an additional optional active caffeic acid ethyl ester in an amount from 5µg/g composition to 7.5µg/g composition, wherein the pharmaceutic carrier comprises at least functional and non-functional topical excipients.

The present invention further relates to a method of reducing and/or treating inflammation by applying topically and/or administering internally a safe and effective amount of one or more arylnaphthalene lignan(s)termed comfreyns as disclosed herein and in particular comfreyn A or a combination of a safe and effective amount of one or more comfreyn(s) and in particular comfreyn A and a safe and effective amount of caffeic acid ethyl ester.

In one embodiment the method is disclosed for
- treating broken bones, tendon damages, painful joints and muscles, acute upper and lower back pain, gonarthrosis, blunt injuries;
- Reducing swelling of joint distortions;
- Improving joint mobility in osteoarthritic joints; and/or combinations thereof, by applying topically and/or administering internally a safe and effective amount of one or more arylnaphthalene lignan(s)termed comfreyns, in particular comfreyn A, or a combination of a safe and effective amount of one or more comfreyn(s), in particular comfreyn A, and a safe and effective amount of caffeic acid ethyl ester.

In another embodiment a method is provided for inhibition and/or treatment of inflammation by interfering (blocking or reducing) with pro-inflammatory gene expression by applying topically and/or administering internally a safe and effective amount of one or more comfreyn(s), in particular comfreyn A, or a combination of a safe and effective amount of one or more comfreyn(s), in particular comfreyn A, and a safe and effective amount of caffeic acid ethyl ester and/or further additional active agents as disclosed herein.

The present invention further relates to a kit comprising a pharmaceutic composition as disclosed herein comprising the one or more arylnaphthalene lignan(s) as disclosed herein, in particular comfreyn A, or one or more arylnaphthalene lignan(s) as disclosed herein, in particular comfreyn A, plus caffeic acid ethyl ester in a safe and effective amount and instructions to use.

### BRIEF DESCRIPTION OF THE DRAWINGS

Fig. 1 shows a LC-MS profile in negative ion mode of a hydroalcoholic liquid extract of *S. officinale* roots;
Fig. 2 shows the structures of the 20 main compounds isolated from the roots of *Symphytum officinale;*
Fig. 3 shows a list of secondary metabolites identified by LC-ESI-HR-MS and LC-ESI-HR-MS/MS from the roots of *Symphytum officinale;*
Fig. 4 shows high resolution MS spectra of comfreyn A shown as compound 15 in Fig. 2; and
Fig. 5 shows the key HMBC (→) and ROESY (↔) correlations of comfreyn A shown as compound 15 of Fig. 2B.

### DETAILED DESCRIPTION OF THE INVENTION

Consumers are looking for effective and natural ways of relieving pain together with inhibiting and reducing inflammation. The arylnaphthalene lignan comfreyn A as disclosed herein was isolated from the roots of *Symphytum officinale* L. and was shown to inhibit the expression of E-selecting mRNA in endothelial cells after stimulation with 5 ng/mL of IL-1β for 90 min. Thereby, a reduction of the inflammatory response is achieved. Thus, the present invention relates to one or more comfreyn(s), pharmaceutic compositions comprising them and their medical use in treatment of inflammatory conditions and diseases, in particular those that are related to pro-inflammatory gene expression.

While the specification concludes with claims particularly pointing out and distinctly claiming the invention, it is believed that the present invention will be understood better from the following description of embodiments, taken in conjunction with the accompanying drawings, in which like reference numerals identify identical elements.

By "cm" as used herein is meant centimeter; by "mm" as used herein is meant millimeter; by "µm" or "microns" as used herein is meant micrometer; by "nm" as used herein is meant nanometer. By "g" as used herein is meant grams; by "mg" as used herein is meant milligrams. By "mL" or "ml" as used herein is meant milliliters; by "µL" or "µl" as used herein is meant microliters. By "min" as used herein is meant minutes, by "sec" as used herein is meant seconds. By "mM" as used herein is meant millimol/liter; by "µM" as used herein is meant micromol/liter. The term "wt%" or "w/w%" as used herein means percentage by weight and generally all percentages and ratios used herein after are by weight of total topical composition unless otherwise indicated.

All percentages, ratios, levels and concentration of ingredients referred to herein are based on the actual amount of the ingredient, and do not comprise solvents, fillers, or other materials with which the ingredient may be combined as a commercially available product, unless otherwise indicated.

All measurements referred to herein are made at about 22°C (i.e. room temperature) unless otherwise specified.

As used herein, the word "about" means +/- 10 percent.

As used herein, the word "or" when used as a connector of two or more elements is meant to include the elements individually and in combination; for example X or Y, means X or Y or both.

As used herein, the articles "a" and "an" are understood to mean one or more of the material that is claimed or described, for example, "an active" or "a solvent".

As used herein, the word "comprise," and its variants, are intended to be non-limiting, such that recitation of items in a list is not to the exclusion of other like items that may also be useful in the materials, oral compositions, devices, and methods of this invention. This term encompasses the terms "consisting of' and "consisting essentially of'.

As used herein, the word "include," and its variants, are intended to be non-limiting, such that recitation of items in a list is not to the exclusion of other like items that may also be useful in the materials, topical compositions, devices, and methods of this invention.

As used herein, the words "preferred", "preferably" and variants refer to embodiments of the invention that afford certain benefits, under certain circumstances. However, other embodiments may also be preferred, under the same or other circumstances. Furthermore, the recitation of one or more preferred embodiments does not imply that other embodiments are not useful and is not intended to exclude other embodiments from the scope of the invention.

"Active", "active agent" and "other ingredients" useful herein may be categorized or described herein by their cosmetic and/or therapeutic benefit or their postulated mode of action or function. However, it is to be understood that the actives and other ingredients useful herein can, in some instances, provide more than one cosmetic and/or therapeutic benefit or function or operate via more than one mode of action. Therefore, classifications herein are made for the sake of convenience and are not intended to limit an ingredient to the particularly stated function(s) or activities listed.

By "safe and effective amount" as used herein is meant an amount of a component, high enough to significantly (positively) modify the condition to be treated or to effect the desired result, but low enough to avoid serious side effects (at a reasonable benefit/risk ratio), within the scope of sound medical judgment. The safe and effective amount of a component, will vary with the particular condition being treated, the age and physical condition of the patient being treated, the severity of the condition, the duration of treatment, the nature of concurrent therapy, the specific form employed, and the particular vehicle from which the component is applied.

As used herein, the terms "administer," "administering," and "administration," refer to any method which, in sound medical practice, delivers the composition to a subject in such a manner as to provide a therapeutic effect.

As used herein, "medication" refers to medications, such as pharmaceuticals, including prescription medications, over-the-counter medications, behind-the-counter medications and combinations thereof. In some examples, a medication can be a supplement.

### Comfreyn(s)

The present invention is directed to one or more arylnaphthalene lignan(s), named hereinafter comfreyn(s). The chemical structure of the one or more comfreyn(s) as claimed is shown in Formula I. 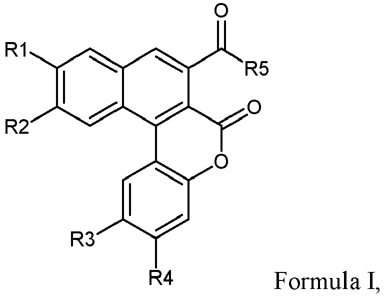 wherein each R1, R2, R3, and R4 are independently selected from hydrogen, hydroxy, methyl, methoxy, ethoxy, propoxy, butoxy or pentoxy; and wherein R5 is selected from hydroxy, methoxy, ethoxy, propoxy, butoxy, pentoxy, feruloyl, caffeoyl, coumaroyl, prenoxy or isoprenoxy residues. The one or more comfreyn(s) as claimed are established as arylnaphtalene lignan(s) bearing an unusual δ-lactone ring, corresponding to a coumarin skeleton and are naturally occurring compounds as natural synthesis pathways exist for all side groups claimed for R1 to R5.

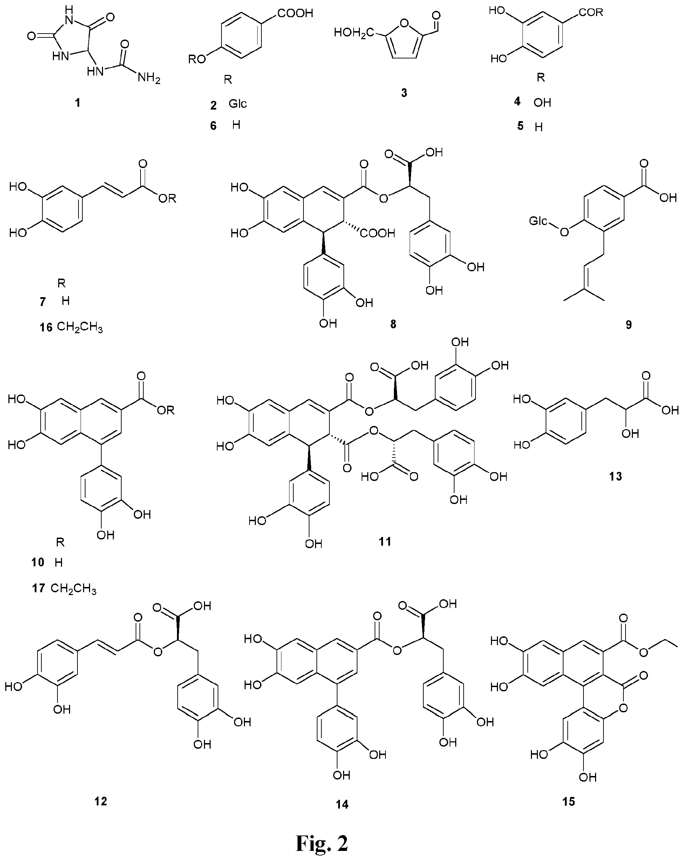

In a preferred embodiment the present invention is directed to an arylnaphtalene lignan, named hereinafter comfreyn A, wherein each of R1, R2, R3, and R4 are hydroxy and wherein R5 is an ethoxy group. The chemical structure is shown in Formula II 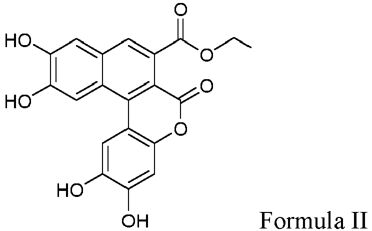 and corresponds to compound 15 of Fig. 2.

In addition, or alternatively, the present invention relates to the therapeutic activity of the one or more comfreyn(s) as claimed herein, in particular to the therapeutic activity of comfreyn A. Comfreyn A was shown to reduce inflammation by interfering with pro-inflammatory gene expression such as inhibition of E-selectin expression in IL-1β stimulated human venous endothelial cells(HUVEC) (Table 2).E-selectin is a NF-κB-dependent target gene and IL-1β is a well-described pro-inflammatory mediator that exerts an important role in the regulation of immune and inflammatory responses, in particular those involving sterile insults such as trauma and blunt injuries. In one embodiment the E-selectin activation is inhibited by at least 30%, preferably by at least 40%, more preferred by at least 45%, more preferred by at least 48%, even more preferred by at least 50% using one or more comfreyn(s) as disclosed herein, in particular using comfreyn A. In addition, or alternatively, the local effective concentration of the one or more comfreyn(s) as disclosed herein, in particular the local effective concentration of comfreyn A to inhibit the in-vitro E-selectin activation, is below 70µM, preferably below 60µM, more preferred about 50µM.

The present invention further relates to inhibition and/or treatment of inflammation using the anti-inflammatory activity of one or more comfreyn(s) as disclosed herein, in particular using the anti-inflammatory activity of comfreyn A via interference with pro-inflammatory gene expression. Without wishing to being bound by theory the anti-inflammatory activity of the one or more comfreyn(s) as disclosed herein, in particular the anti-inflammatory activity of comfreyn A based on dampening pro-inflammatory gene expression may be used for the treatment of broken bones, tendon damages, painful joints and muscles, acute upper and lower back pain, gonarthrosis, blunt injuries, for reduction of swelling of joint distortions, for improvement of joint mobility in osteoarthritic joints and/or combinations thereof by applying the one or more comfreyn(s), in particular by applying comfreyn A in a safe and effective amount, preferably in an amount to provide a local concentration from about 30µM to about 65µM, more preferred in an amount to provide a local concentration from about 40µM to about 60µM, more preferred in an amount to provide a local concentration from about 45µM to about 55µM, even more preferred in an amount to provide a local concentration from 48µM to 52µM comfreyn A at the location in need thereof.

In a preferred embodiment, a suitable composition comprises comfreyn A in an amount from 1µg/g of composition to 10µg/g of composition, in particular in an amount from 2µg/g of composition to 7.5µg/g of composition, and even more particular in an amount from 3µg/g of composition to 5µg/g of composition, wherein a suitable single dose is from 3g to 5g composition and application at least three times a day is recommended.

### Additional active agents

In addition, or alternatively and according to the invention as disclosed herein, one or more comfreyn active agent(s), in particular comfreyn A, may be combined with additional active agents. A suitable additional active agent according to the present invention is preferably a pain reliver, an active agent with anti-inflammatory activity, an activity enhancer or a combination thereof. In a preferred embodiment, the anti-inflammatory activity of the additional active agent via interfering with pro-inflammatory gene expression as disclosed herein is inhibited by at least 30%, preferably by at least 40%, more preferred by at least 45%, more preferred by at least 48%, even more preferred by at least 50%. In addition, or alternatively, the effective concentration of the additional active agent to inhibit pro-inflammatory gene expression, is below 70µM, preferably below 60µM, more preferred below 50µM, more preferred below 40µM, more preferred below 30µM and even more preferred below 25µM as shown by in-vitro experiments.

Non-limiting examples of chemical pain relievers can include APAP, ibuprofen, ketoprofen, diclofenac, naproxen, aspirin, and combinations thereof. In one example from about 0.5% to about 3.5% by weight of the composition pain reliever may be used as additional active agent, in another example from about 1% to about 3% pain reliever, and in another example from about 1.5% to about 2% pain reliever, by weight of the composition may be used. For example, a dose can comprise 325 mg to 500 mg APAP, 200 mg ibuprofen, 200 mg naproxen or a combination thereof.

In a preferred embodiment, the additional active agent is a natural pain reliever, in particular a natural pain reliver extracted from *Symphytum officinale* L., more particular extracted from *Symphytum officinale* L. roots. Without wishing to being bound by a theory it is believed that the combination of more than one active agents, in particular of more than one active agent extracted from *Symphytum officinale* L. roots may increase the anti-inflammatory activity. In a preferred embodiment, one or more comfreyn(s), in particular comfreyn A is combined with one or more additional active agent(s), selected from allantoin, *p*-hydroxy benzoic acid glucoside, 5-hydroxymethyl-2-furfural, protocatechuic acid, protocatechuic aldehyde, *p*-hydroxy benzoic acid, caffeic acid, globoidnan B, malaxinic, acid, 3-carboxy-6,7-dihydroxy-1-(3',4'-dihydroxyphenyl)-naphthalene, (+)-rabdosiin, rosmarinic acid, hydroxy caffeic acid,globoidnan A, caffeic acid ethyl ester, ternifoliuslignan D, linolenic acid, linoleic acid and hydroxypalmitic acid. In particular, comfreyn A is combined with one or more additional active agent(s) selected from globoidnan B, malaxinic acid, globoidnan A, caffeic acid ethyl ester, ternifoliuslignan D, 3-carboxy-6,7-dihydroxy-1-(3',4'-dihydroxyphenyl)-naphthalene and rabdosiin.

In a preferred embodiment, one or more comfreyn(s), in particular comfreyn A are combined with one or more additional active agent(s) comprising or consisting of caffeic acid ethyl ester, malaxinic acid and globoidnan A or a combination thereof. More preferred, comfreyn A is combined with caffeic acid ethyl ester. In one embodiment the pro-inflammatory gene expression is inhibited by at least 30%, preferably by at least 40%, more preferred by at least 50%, more preferred by at least 60%, more preferred by at least 65% and even more preferred by 70% using caffeic acid ethyl ester. In addition, or alternatively, the effective concentration of caffeic acid ethyl ester to inhibit pro-inflammatory gene expression, is below 80µM, preferably below 70µM, more preferred below 65µM, as shown by in vitro experiments.

Caffeic acid ethyl ester can be used in a safe and effective amount as active agent in a pharmaceutic composition as disclosed herein. Without wishing to being bound by a theory the anti-inflammatory activity of caffeic acid ethyl ester is achieved via inhibition of pro-inflammatory gene expression and thus, the present invention relates to inhibition and/or treatment of inflammation, preferably for the treatment of broken bones, tendon damages, painful joints and muscles, acute upper and lower back pain, gonarthrosis, blunt injuries, for reduction of swelling of joint distortions, for improvement of joint mobility in osteoarthritic joints and/or combinations thereof by applying caffeic acid ethyl ester in a safe and effective amount, preferably in an amount provide a local concentration from about 50µM to about 80µM, more preferred in an amount provide a local concentration from about 55µM to about 75µM, more preferred in an amount provide a local concentration from about 60µM to about 70µM, even more preferred in an amount provide a local concentration from 62µM to 66µM caffeic acid ethyl ester at the location in need thereof.

In a preferred embodiment a suitable composition comprises caffeic acid ethyl ester in an amount from 1µg/g of composition to 15µg/g of composition, in particular in an amount from 25µg/g of composition to 10µg/g of composition, and even more particular in an amount from 5µg/g of composition to 7.5µg/g of composition, wherein a suitable single dose is from 3g to 5g composition and application at least three times a day is recommended.

### Pharmaceutic composition(s)

The present invention relates to a pharmaceutic composition comprising one or more arylnaphthalene lignan(s) as disclosed herein, in particular comfreyn A in a safe and effective amount as disclosed herein and a pharmaceutically acceptable carrier. The term "pharmaceutically acceptable carrier" as used herein comprises one or more compatible solid or semi-solid or liquid excipients or diluents which are suitable for topical or internal administration. By "compatible," as used herein, is meant that the components of the pharmaceutic composition are capable of being commingled without interaction in a manner which would substantially diminish the composition's stability and/or efficacy.

The choice of pharmaceutic excipient, carrier, or diluent is selected with regard to the intended route of administration and standard pharmaceutic practice. Examples of such suitable excipients for the various different compositions described herein in some aspects are found in the "Handbook of Pharmaceutical Excipients, 2nd Edition, (1994), Edited by A Wade and P J Weller. Acceptable carriers in some aspects are described, for example, in Remington's Pharmaceutical Sciences, Mack Publishing Co. (A. R. Gennaro edit. 1985). Such compositions may comprise additional ingredients in addition to the excipient, carrier or diluent. Such additional ingredients include, but are not limited to, any suitable binder(s), lubricant(s), coating agent(s), solubilizing agent(s), emulsifier(s), surfactant(s), preservatives, dyes, flavoring agent(s), stabilizing agent(s), buffering agent(s)and/or suspending agent(s). In addition, such formulation may further include a penetration enhancer, or any other functional or non-functional excipient.

### Topical composition(s)

The pharmaceutic compositions as disclosed herein are useful for topical application, in particular for topical application at the skin, i.e. by a "topical composition" as used herein is meant a product which in the ordinary course of usage is not used for purposes of systemic administration of therapeutic agents, but is applied to and retained at the skin for a sufficient time to achieve intended purposes, e.g. inhibition of inflammation, pain relief etc..

Depending on the type and amount of ingredients therein, the desired consistency and the contemplated mode of application of the topical formulation, the carrier material may include carrier materials such as pharmacologically-acceptable oils, water and/or such supplementary pharmaceutical adjuvants which are conventionally used in topical formulations, e.g., in conventional bases for ointments, creams, and gels. In some embodiments it may be advisable to incorporate a structure-forming, thickening or gel-forming agent into the composition. Suitable agents are, in particular, highly dispersed silicic acid (e.g. the commercial product "Aerosil"), bentonites, modified montmorillonites such as alkyl ammonium salts of montmorillonites (e.g. the commercial product "Bentone"), wherein the alkyl groups may contain 1 to 20 carbon atoms, e.g. dimethyl-dialkyl-ammonium salts wherein the alkylgroups contain 16 to 18 carbon atoms, organic structure forming, thickening and suspending agents, e.g. xanthan gum, carrageenan gum, cetostearylic alcohol, and/or modified castor oil products. In addition, or alternatively, such compositions may comprise additional ingredients in addition to the excipient, carrier or diluent. Such additional ingredients may include, but are not limited to any suitable binder(s), lubricant(s), coating agent(s), solubilizing agent(s), solvent(s), emulsifier(s), lipid(s), polymer(s), gelling agent(s), surfactant(s), penetration enhancer (s), pH modifier(s), buffer(s), preservatives, dye(s), flavoring agent(s), stabilizing agent(s), buffering agent(s) and/or suspending agent(s) or fragrance(s) or chelating agent(s).

The method of applying the active ingredients separately or as combined components in the topical formulation as disclosed herein will typically involve application to an area affected by inflammation and/or degeneration, or an area expected to become affected by inflammation and/or degeneration, or to an area affected or expected to become affected by pain, swelling, stiffness and/or lack of mobility associated with said inflammation and/or degeneration. In a certain embodiment, the active ingredients are applied to an area that surrounds the affected site or to an area expected of becoming affected. For instance, the active ingredients may be applied separately, or as combined components in the topical formulation of the present invention, to an area that is all around the affected area or expected area of inflammation and/or degeneration. The topical formulation will include, but is not limited to cream, gel, ointment or lotion, and it may be spread on the body surface and gently rubbed in. Types of inflammatory and/or degenerative conditions applicable to the methods and uses of the present invention include, but are not limited to, rheumatoid arthritis, septic arthritis, gout, pseudogout, juvenile arthritis, Still's disease, cervical spondylitis, lumbar spondylitis, frozen shoulder, calcaneal spurs, psoriatic arthritis, ankylosing spondylitis and osteoarthritis.

In a certain embodiment of the present invention, the subject for application is human. However, the methods of prophylactic or therapeutic treatment in accordance with the present invention may also be applicable to non-human subjects that are susceptible to inflammatory and/or degenerative conditions, including, but not limited to, horses, dogs, cats, rabbits and/or bovine.

The dose regimen will depend on a number of factors that may readily be determined, such as the extent of inflammation and/or degeneration or pain, swelling, stiffness and/or lack of mobility associated with said inflammation and/or degeneration and the responsiveness of the inflammation, degeneration, pain, swelling, stiffness and/or lack of mobility to treatment, but will normally be one or more doses per day, with a course of treatment lasting from several days to several months, or until a cure is effected or a significant diminution in the extent of the inflammation, degeneration, pain, swelling, stiffness and/or lack of mobility experienced by the subject. One of ordinary skill may readily determine optimum dosages, dosing methodologies and repetition rates. In a certain embodiment, it is contemplated that the topical formulation will be applied one to five times daily.

By "a sufficient amount of composition to provide an effect" as used herein is meant that an amount of the topical composition is applied to, used or worn by the patient or the patient is instructed to apply, use or wear an amount of the topical composition of greater than about 1g, greater than about 2g, greater than about 3g, preferably about 4g, in particular from about 1, 2, 3, 3.5 or 4g to about 10, 8, 6, 5, or 4.5g per application or any other numerical range which is narrower and which falls within such broader numerical range, as if such narrower numerical ranges were all expressly written herein. The treatments may be applied by need and will depend on the desired effect, the severity of any condition being treated, the health and age of the user and like considerations. In general application of three times per day is recommended. In one embodiment the one or more actives, preferably the one or more comfreyn active(s), in particular the comfreyn A may be used in an amount from 1µg/g of composition to 10µg/g of composition, preferably in an amount from 2µg/g of composition to 7.5µg/g of composition, and more preferably in an amount from 3µg/g of composition to 5µg/g of composition.

The topical formulations according to the present invention may be prepared in any conventional manner though not limited to the known pharmaceutical practices. The topical formulation as disclosed herein may be aqueous (i.e., containing water), or it may be nonaqueous and optionally used in combination with an occlusive overlayer so that moisture evaporating from the body surface is maintained within the formulation upon application to the body surface and thereafter. Further, the formulation can be applied directly with or without the need for a bandage or pressure pad to be applied. The present compositions could also include for example; leave-on compositions, wherein "leave-on" as used herein, means compositions intended to be applied to and allowed to remain on the keratinous tissue. These leave-on compositions are to be distinguished from compositions which are applied to the skin and subsequently (in a few minutes or less) removed either by washing, rinsing, wiping, or the like. Leave-on compositions exclude rinse-off applications such as shampoos, facial cleansers, hand cleansers, body wash, or body cleansers.

Thus, the topical compositions as disclosed herein are "dermatologically acceptable" which means that the compositions or components described are suitable for use in contact with human skin tissue without undue toxicity, incompatibility, instability, allergic response, and the like. The topical pharmaceutic composition as disclosed herein may be for example in the form of an ointment, a salve, a paste, a viscous liquid, a lotion, a gel, a cream, a mousse, a foam, or any other adhesive product or a combination thereof.

The topical composition may be a single-phase composition or may be a combination of two or more separate compositions, wherein two or more separate compositions may be contained in physically separated compartments of a dispenser and dispensed side-by-side. The term "dispenser", as used herein, means any pump, tube, or container suitable for dispensing topical compositions.

The topical composition may also be incorporated onto a delivery carrier for direct application or attachment to skin surfaces. The term "delivery carrier" as used herein comprises a material or an appliance, but is not limited to those mentioned, that is used to hold the topical composition against the skin surface. Delivery carriers may be for example strips or a polymeric film or a backing membrane or a patch. These delivery carriers may be rectangular, arched, curved, semi-circular, have rounded corners, have slits cut into it, have notches cut into it, bent into three dimensional shapes, or combinations thereof. They may be solid, semi-solid, textured, moldable, flexible, deformable, permanently deformable, or combinations thereof. Strips may be made from for example plastic sheets including polyethylene, or wax sheets or polymeric carriers. The size of these is adapted according to the area to be treated.

In addition, or alternatively, the topical formulation of the present invention may also be administered through the skin or mucosal tissue using a conventional skin patch, wherein the active agent(s) are contained within a laminated structure that serves as a delivery device to be affixed to the body surface. In such a structure, the topical formulation is contained in a layer, or "reservoir," underlying an upper backing layer. The laminated structure may contain a single reservoir, or it may contain multiple reservoirs. In one embodiment, the reservoir comprises a polymeric matrix of a pharmaceutically acceptable adhesive material that serves to affix the system to the skin during delivery of the topical formulation. Typically, the adhesive material is a pressure-sensitive adhesive (PSA) that is suitable for long-term skin contact, and that should be physically and chemically compatible with the topical formulation. Examples of suitable adhesive materials include but are not limited to: polyethylenes; polysiloxanes; polyisobutylenes; polyacrylates; polyacrylamides; polyurethanes; plasticized ethylene-vinyl acetate copolymers; and tacky rubbers such as polyisobutene, polybutadiene, polystyrene-isoprene copolymers, polystyrene-butadiene copolymers, and neoprene (polychloroprene). Preferred adhesives are polyisobutylenes. The backing layer functions as the primary structural element of the transdermal system and provides the device with flexibility and, preferably, occlusivity. The material used for the backing layer should be inert and incapable of absorbing any one or more of the active agents included in the topical formulation contained within the device. The backing is preferably comprised of a flexible elastomeric material that serves as a protective covering to prevent loss of drug and/or vehicle via transmission through the upper surface of the patch, and will preferably impart a degree of occlusivity to the system, such that the area of the body surface covered by the patch becomes hydrated during use. The material used for the backing layer should permit the device to follow the contours of the skin and be worn comfortably on areas of skin such as at joints or other points of flexure, that are normally subjected to mechanical strain, with little or no likelihood of the device disengaging from the skin due to differences in the flexibility or resiliency of the skin and the device. The materials used as the backing layer are either occlusive or permeable, as noted above, although occlusive backings are preferred, and are generally derived from synthetic polymers (e.g., polyester, polyethylene, polypropylene, polyurethane, polyvinylidine chloride, and polyether amide), natural polymers (e.g., cellulosic materials), or macroporous woven and nonwoven materials.

In addition, or alternatively, the topical formulation-containing reservoir and skin contact adhesive are present as separate and distinct layers, with the adhesive underlying the reservoir, wherein the reservoir may be a polymeric matrix as described above. Alternatively, the reservoir may be comprised of a liquid or semisolid topical formulation contained in a closed compartment or "pouch," or it may be a hydrogel reservoir, or may take some other form.

Additional layers e.g., intermediate fabric layers and/or rate-controlling membranes, may also be present in any of these delivery systems. Fabric layers may be used to facilitate fabrication of the device, while a rate-controlling membrane may be used to control the rate at which the topical formulation permeates out of the device.

A rate controlling membrane, if present, will be included in the system on the skin side of one or more of the reservoirs. The materials used to form such a membrane are typically selected to limit the flux of one or more components contained in the topical formulation. Representative materials useful for forming rate-controlling membranes include polyolefins such as polyethylene and polypropylene, polyamides, polyesters, ethylene-ethacrylate copolymer, ethylene-vinyl acetate copolymer, ethylene-vinylmethylacetate copolymer, ethylene-vinyl ethylacetate copolymer, ethylene-vinylpropylacetate copolymer, polyisoprene, polyacrylonitrile, ethylene-propylene copolymer, and the like.

Generally, the underlying surface of the transdermal device (i.e. the skin contact area) has an area in the range of about 0.25 cm2 to 200 cm2, preferably 1 cm2 to 25 cm2, more preferably 2 cm2 to 10 cm2. That area will vary, of course, with the size of the area to be treated.

Delivery systems as disclosed herein may be fabricated using conventional coating and laminating techniques known in the art. For example, adhesive matrix systems can be prepared by casting a fluid admixture of an adhesive and the topical formulation of the present invention onto the backing layer, followed by lamination of the release liner. Similarly, the adhesive mixture may be cast onto the release liner, followed by lamination of the backing layer. Alternatively, the reservoir may be prepared in the absence of topical formulation, and then loaded by "soaking" in the topical formulation. In general, these patches are fabricated by solvent evaporation, film casting, melt extrusion, thin film lamination, die cutting, or the like.

The topical formulation will generally be incorporated into the device during patch manufacture rather than subsequent to preparation of the device. The delivery system may also include an adhesive overlayer that also serves as a backing for the delivery system is used to better secure the patch to the body surface. This overlayer is sized such that it extends beyond the reservoir so that adhesive on the overlayer comes into contact with the body surface. The overlayer is useful because the adhesive/reservoir layer may lose its adhesion a few hours after application due to hydration. By incorporating such adhesive overlayer, the delivery system remains in place for the required period of time.

### Topical carrier

The topical formulations according to the present invention may take the form of compositions suitable for topical application to the body surface, and may comprise, for example, a cream, lotion, solution (e.g., liquid spray), gel, film, ointment, paste, plaster, paint, bioadhesive, or the like.

### Ointments

Ointments, as it is well known in the art, are semisolid preparations that are typically based on petrolatum or other petroleum derivatives. The specific ointment base to be used, as will be appreciated by those skilled in the art, is one that will provide for optimum delivery of the active agent(s), and which may also provide for other desired characteristics as well, such as emolliency or the like. As with other carriers or vehicles, an ointment base is typically inert, stable, nonirritating and nonsensitizing. Ointment bases may be grouped in four classes: oleaginous bases; emulsifiable bases; emulsion bases; and water-soluble bases. Oleaginous ointment bases include, for example, vegetable oils, fats obtained from animals, and semisolid hydrocarbons obtained from petroleum. Emulsifiable ointment bases, also known as absorbent ointment bases, contain little or no water and include, for example, hydroxystearin sulfate, anhydrous lanolin and hydrophilic petrolatum. Emulsion ointment bases are either water-in-oil (w/o) emulsions or oil-in-water (o/w) emulsions, and include, for example, cetyl alcohol, glyceryl monostearate, lanolin, stearic acid and polyethylene glycols of varying molecular weight, or combinations thereof. Other examples of suitable hydrocarbon bases include, but are not limited to, hard, soft, or liquid paraffin, glycerol, beeswax, metallic soap, a mucilage, an oil of natural origin (such as almond, corn, arachis, castor or olive oil), wool fat or its derivative, a fatty acid (such as stearic acid or oleic acid), or combinations thereof. All of the above ingredients that contribute to the process of making the ointments are not limited to their synthetic nature and could be natural or of natural origin or naturally derived.

### Creams

Creams as also well known in the art, are viscous liquid or semisolid oil-in-water emulsions. Cream bases can be natural or synthetic water-washable, and contain an oil phase, an emulsifier, and an aqueous phase. The oil phase, also called the "internal" phase, may include petrolatum and a fatty alcohol such as cetyl or stearyl alcohol. The aqueous phase usually, although not necessarily, exceeds the oil phase in volume, and generally contains a humectant. The emulsifier in a cream formulation is generally a nonionic, anionic, cationic, or amphoteric surfactant, or combinations thereof. Further to this, novel delivery techniques as incorporated in cream bases could also be included thereof. All of the above ingredients that contribute to the process of making the creams are not limited to their synthetic nature and could be natural or of natural origin or naturally derived.

### Gels

Gel or emulgel formulations are typically semisolid, suspension-type systems. Single-phase gels typically contain organic macromolecules distributed substantially uniformly throughout the carrier liquid, which is typically aqueous, but may also contain an alcohol and, optionally, an oil. Exemplary "organic macromolecules" (i.e., gelling agents), are crosslinked acrylic acid polymers such as the "carbomer" family of polymers (e.g., carboxypolyalkylenes that may be obtained commercially under the Carbopol^{™}). Also exemplified are hydrophilic polymers such as polyethylene oxides, polyoxyethylene-polyoxypropylene copolymers, polyvinylalcohol and polyvinyl pyrrolidones; cellulosic polymers such as hydroxypropyl cellulose, hydroxyethyl cellulose, hydroxypropyl methylcellulose, carboxymethyl cellulose, hydroxypropyl methylcellulose phthalate, and methyl cellulose; gums such as carrageenan gum, tragacanth and xanthan gum; sodium alginate; and gelatin. Examples of suitable gel forming agents include carboxypoly-methylene derivatives such as carbopol and pectins. In one embodiment of the present invention, the gelling agent is xanthan gum and/or carrageenan gum.

In one embodiment, the thickening or gelling agents may be present in the composition generally in a w/w % amount of from about 0.2% to 5.0%. In another embodiment, the thickening or gelling agents may be present in the composition generally in a w/w % amount of from about 0.1% to 1.0%. In yet another embodiment of the present invention, the topical gel formulation may include a gelling agent in an amount of up to 3.0% w/w (e.g. xanthan gum).

In order to prepare a uniform gel, a diluent such as alcohol can be added, or the gelling agent can be dispersed by trituration, mechanical mixing or stirring, or combinations thereof. Furthermore the gel or emulgel formulations may contain other auxiliary agents commonly used in the kind of formulations like preservatives, antioxidants, colors, fragrances, stabilizers etc..

Hydrogels are macromolecular networks that absorb water and thus swell but do not dissolve in water. That is, hydrogels contain hydrophilic functional groups that provide for water absorption, but the hydrogels are comprised of crosslinked polymers that give rise to aqueous insolubility. Generally, then, hydrogels are comprised of crosslinked hydrophilic polymers such as a polyurethane, a polyvinyl alcohol, a polyacrylic acid, a polyoxyethylene, a polyvinylpyrrolidone, a poly(hydroxyethyl methacrylate) (poly(HEMA)), or a copolymer or mixture thereof. Particularly preferred hydrophilic polymers are copolymers of HEMA and polyvinylpyrrolidone.

All of the above ingredients that contribute to the process of making the gel or emulgel are not limited to their synthetic nature and could be natural or of natural origin or naturally derived.

### Lotions

Lotions are typically preparations to be applied to the skin surface without friction and are typically liquid or semiliquid preparations in which solid particles, including the active agent(s) are present in a water or alcohol base. Lotions are usually suspensions of solids, and may, for the present purpose, comprise a liquid oily emulsion of the oil-in-water type. Lotions can be used to treat large body areas because of the ease of applying a more fluid composition. It is generally desirable that the insoluble matter in a lotion be finely divided. Thus, lotions will typically contain suspending agents to produce better dispersions, as well as compounds useful for localizing and holding the active agent in contact with the skin, e.g. methylcellulose, sodium carboxymethyl-cellulose, or the like. Lotions may also include an agent to hasten drying and cooling of the solution on the skin, such as alcohol or acetone. They may further include a moisturizer, such as glycerol, or an oil, such as castor oil or arachis oil.

Topical formulations of the present invention may also be prepared with liposomes, and/or micelles. Liposomes are microscopic vesicles having a lipid wall comprising a lipid bilayer and can be used as delivery systems herein. Generally, liposome formulations are employed for poorly soluble or insoluble agents. Liposomal preparations for use in the present invention may include cationic (positively charged), anionic (negatively charged) and neutral preparations. Cationic liposomes are readily available. For example, N[1-2,3-dioleyloxy)propyl]-N,N,N-triethylammonium (DOTMA) liposomes are available under the tradename Lipofectin^{™}. Similarly, anionic and neutral liposomes are readily available as well or can be easily prepared using readily available materials. Such materials include phosphatidyl choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with DOTMA in appropriate ratios. Methods for making liposomes using these materials are well known in the art.

Micelles, in an aqueous solution, are known in the art to be comprised of surfactant molecules arranged so that their polar headgroups form an outer spherical shell, while the hydrophobic, hydrocarbon chains are oriented towards the center of the sphere, forming a core. Micelles form in an aqueous solution containing a surfactant at a high enough concentration so that micelles naturally result. Surfactants useful for forming micelles include, but are not limited to, potassium laurate, sodium octane sulfonate, sodium decane sulfonate, sodium dodecane sulfonate, sodium lauryl sulfate, docusate sodium, decyltrimethylammonium bromide, dodecyltrimethylammonium bromide, tetradecyltrimethylammonium bromide, tetradecyltrimethylammonium chloride, dodecylammonium chloride, polyoxyl 8 dodecyl ether, polyoxyl 12 dodecyl ether, nonoxynol 10 and nonoxynol 30. Micelle formulations can be used in conjunction with the present invention either by incorporation into the reservoir of a topical delivery system, or into a topical formulation to be applied to the body surface. Various additives, known to those skilled in the art, may also be included in the topical formulations of the present invention.

In addition, or alternatively, solvents, including relatively small amounts of alcohol, may be used to solubilize certain formulation components. Suitable solvents include glycerin, SD 40 alcohol, lecithin, poloxamers, Miglyol^{™} and methyl pyrrolidone, or combinations thereof. Other suitable solvents include glycerol, diluted alcohol isopropanol, hexylene glycol and propylene glycol. Menthol may be also be added to the topical formulation of the present invention. In one embodiment, surfactant(s) may be employed in the topical formulation according to the present invention, for example, in an amount of from about 0.5% to about 5.0% w/w. Suitable surfactants may include, but are not limited to sodium lauryl sulfate, benzalkonium chloride, cetylpyridinium chloride, sodium laurate, cetyltrimethylammonium bromide, Poloxamer (231, 182, 184), Tween (20, 40, 60, 80) and Span^{™} and combinations thereof.

The formulations may also contain irritation-mitigating additives to minimize or eliminate the possibility of skin irritation or skin damage resulting from the pharmacologically active base or other components of the composition. Suitable irritation-mitigating additives include, for example: α-tocopherol; monoamine oxidase inhibitors, particularly phenyl alcohols such as 2-phenyl-1 -ethanol; glycerin; ascorbic acids and ascorbates; ionophores such as amphiphilic amines; ammonium chloride; N-acetylcysteine; cis-urocanic acid; capsaicin.

In another embodiment of the present invention, the delivery system may include nanoparticles. The nanoparticles may be dispersed within a microemulsion in the formulation of the present invention, or as part of the vehicle of such formulations, for use in delivery of the active agent(s) through the skin. Without wishing to being bound by theory, such nanoparticles will readily penetrate the skin and may be retained within specific layers of the skin. In one embodiment, the nanoparticles have a core comprised of an iron ore that is strongly attracted by a magnet. In addition, or alternatively, the nanoparticles include magnetite, which in one embodiment refers to a molecule with a general formula of Fe3O4. The nanoparticles may also include chemical equivalents of magnetite, such as, for example (Fe M)OFe2O3 where M may be, in one embodiment, Zn, Co, Ni, Mn, Cr, Au, or Ag. The nanoparticles for use in the delivery system and according to the methods of this invention may e.g. range in size from 1-100 nm. Polymers may also be conjugated to the nanoparticles by an array of means well known by those skilled in the art. The active agent(s), in turn, may be conjugated to the polymer, conjugated to the nanoparticle or, as a function of the physicochemical properties of the compound, may be directly affixed to the nanoparticle. The choice of polymer utilized may be a function of the particles employed. In one embodiment, the polymer includes polyacrylic acid, polystyrene sulfonic acid, polyvinyl sulfonic acid, polyethylene oxide, polypropylene oxide, polyvinyl alcohol, photopolymerizable macromers, biodegradable polymers such as but not limited to PLGA, or a combination thereof. In another embodiment, the polymer includes a surfactant, a polyethylene glycol, a lignosulfonate, a polyacrylamide or a biopolymer. In yet another embodiment, the biopolymer includes polypeptides, cellulose and its derivatives such as hydroxyethyl cellulose and carboxymethyl cellulose, alginate, chitosan, lipid, dextran, starch, gellan gum or other polysaccharides, or a combination thereof.

The topical formulations of the present invention may also include other compositions that facilitate the delivery of the active ingredient(s) through the skin. For example, the active agents(s) may be formulated into macromolecular assemblies using nanoparticles, as described, for example, in WO2006/113668, WO2006/129127 and US60/671,892 as published (the entire contents of which are incorporated herein by reference). Without wishing to being bound by theory, macromolecular assemblies of this type typically mimic natural lipoprotein and are therefore said to solubilize hydrophobic active agents to facilitate their delivery (e.g. transdermal delivery) by penetrating the inter-corneocyte lipid layer of the skin and also the hydrophilic pores. Without wishing to being bound by theory, the macromolecular assemblies may become trapped within the stratum corneum of the skin and therefore act as reservoirs for the active agent(s) to enable sustained release into the deeper layers of the skin and thereby provide a distinct therapeutic profile. This could in turn enhance the efficacy of the active agent(s), reduce the number of applications required and quantity of active agent(s) required, and could therefore be more convenient for the patient.

These and other types and configurations of topically applied delivery systems may be used alone or in combination to facilitate the delivery of the active agent(s) included in the formulations of the present invention, as will be appreciated by those skilled in the art of, for example, topical drug delivery. Thus, it is to be understood that the delivery system may include any single or combinations of the embodiments listed herein.

Any suitable emollient or skin conditioning agent may optionally be included in the topical formulations of this invention. Suitable emollients include, but are not limited to, cholesterol, glycerine, glyceryl monostearate, isopropyl myristate, isopropyl palmitate, cetostearyl alcohol, lanolin alcohols and mixtures thereof. Optionally, dimethicone, mineral oil or white soft paraffin may also be incorporated into the formulations in relatively small amounts to act as a skin conditioner. The emollient or skin conditioning agent may be present in the topical formulations of this invention in a w/w % amount of from about 0.5% to about 50%. For example, in the gel formulations of this invention, the emollient or skin conditioning agent may generally be present in an amount of up to about 50% w/w. In another embodiment, the cream or lotion formulations of this invention may include the emollient or skin conditioning agent in an amount of from about 0.5% to about 15% w/w.

The formulations of the present invention may optionally include a buffer or neutralizing agent. Examples of suitable buffers include, but are not limited to citric acid, lactic acid, oleic acid, sodium phosphate, water, triethanolamine, sodium citrate, hydrochloric acid and the like. The buffering agent may be present in the composition in any suitable buffering effective amount. The gel formulation will generally contain a base, such as for example, sodium hydroxide, triethanolamine and the like. The gel formulations of this invention will also generally include a volatile solvent, such as for example, ethanol, isopropanol and the like.

Other agents may also be added to the topical formulation of the present invention including antimicrobial agents, to prevent spoilage upon storage (i.e. to inhibit growth of microbes such as yeasts and molds. Suitable antimicrobial agents are typically selected from the group consisting of the methyl and propyl esters of p-hydroxybenzoic acid (i.e. methyl and propyl paraben), sodium benzoate, sodium hydroxymethyl glycinate, sorbic acid, imidurea, potassium iodide and combinations thereof. The list is not limited to synthetic materials but could also be natural or naturally derived or from natural origin.

The topical formulation of the present invention may optionally include preservative or antioxidant components not limited to natural or synthetic source. Examples of such preservative and antioxidant include, but are not limited to alkyl alcohols, benzyl alcohol, butylated hydroxyanisole, butylated hydroxytoluene (methyl and propyl paraben), butyl paraben, disodium edetate, citric acid, grape seed fruit extracts, sesamol and the like. The preservative and/or antioxidant may be present in the formulations of this invention in a w/w amount of from about 0.05 to about 5.0% w/w. In another embodiment, the preservative and/or antioxidant may be present in the formulations of this invention in an amount of from about 0.25% to about 0.5% w/w.

In certain embodiments, the topical formulation of the present invention may also include other active ingredients including, but not limited to eucalyptus oil (e.g. eucalyptus globulus oil, Eucalyptus tereticortis oil, Eucalyptus rostrata), cineole or other terpenes derived from eucalyptus oil, boswellic acid, glycerin, kelp, potassium iodate, algin, lavender, 1,8-cineole, linalol, lynalol acetate, nut gall extract, lecithin, liquorice extract (e.g., liquorice root extract), glabridin, glycyrrhizic acid, glycyrrhizic acid monoammonium salt, 10-beta-glycyrrhetinic acid, ellagic acid, turmeric, curcumin, tetrahydro curcuminoid (THC), or any combination thereof. Without wishing to being bound by theory, the other active ingredient(s) may contribute, at least in part, to the efficacy of the formulation of the present invention by acting directly or indirectly on a physiological or pathophysiological pathway.

Other base carriers in the topical compositions as disclosed herein comprise a base carrier material. A suitable base carrier material are non-toxic oil(s), non-toxic edible oil(s), saturated or unsaturated fatty alcohol(s), aliphatic hydrocarbon(s), long chain triglyceride(s), fatty ester(s), and mixtures thereof. In addition, or alternatively, the hydrophobic phase may also comprise silicone(s), polysiloxane(s), and mixtures thereof.

Suitable aliphatic hydrocarbons may contain from about 10, 12, 14, or 16 to about 16, 18, 20, 22, 24, 26, 28, 30, 36, 40 carbon atoms such as decane, 2 ethyldecane, tetradecane, isotetradecane, hexadecane, eicosane, and mixtures thereof. Long chain triglycerides include vegetable oils, fish oils, animal fats, hydrogenated vegetable oils, partially hydrogenated vegetable oils, semi-synthetic triglycerides, synthetic triglycerides, and mixtures thereof. Fractionated, refined or purified oils of these types can also be used. Specific examples of suitable long chain triglyceride-containing oils suitable for use in the compositions of the present invention include almond oil; babassu oil; borage oil; black currant seed oil; canola oil; castor oil; coconut oil; corn oil; cottonseed oil; emu oil; evening primrose oil; flax seed oil; grapeseed oil; groundnut oil; mustard seed oil; olive oil; palm oil; palm kernel oil; peanut oil; rapeseed oil; safflower oil; sesame oil; shark liver oil; soybean oil; sunflower oil; hydrogenated castor oil; hydrogenated coconut oil; hydrogenated palm oil; hydrogenated soybean oil; hydrogenated vegetable oil; a mixture of hydrogenated cottonseed oil and hydrogenated castor oil; partially hydrogenated soybean oil; a mixture of partially hydrogenated soybean oil and partially hydrogenated cottonseed oil; glyceryl trioleate; glyceryl trilinoleate; glyceryl trilinolenate; a Q3-polyunsaturated fatty acid triglyceride containing oil; and mixtures thereof. The long chain triglyceride containing oils may be in particular selected from the group consisting of corn oil, olive oil, palm oil, peanut oil, safflower oil, sesame oil, soybean oil, castor oil, linseed oil, rape oil, rice bran oil, coconut oil, hydrogenated castor oil; partially hydrogenated soybean oil; glyceryl trioleate; glyceryl trilinoleate; a Q3-polyunsaturated fatty acid triglyceride containing oil; and mixtures thereof.

Suitable saturated or unsaturated fatty alcohols have from about 6 to about 20 carbon atoms, cetearyl alcohol, lauryl alcohol, and mixtures thereof. For example, Lipowax (Cetearyl Alcohol and Ceteareth-20) are supplied and manufactured by Lipo Chemical.

A suitable mineral oil is petrolatum and mixtures thereof. Examples of petrolatum include Snow White Pet - C from Calumet Specialty Products (Indianapoli, IN), G-2191 from Sonneborn (Parsippany, NJ), G-2218 from Sonneborn, G-1958 from Sonneborn, and mixtures thereof.

General information on silicones including silicone fluids, gums and resins, as well as the manufacture of silicones, can be found in Encyclopedia of Polymer Science and Engineering, Volume 15, Second Edition, pp 204-308, John Wiley & Sons Inc. 1989 and Chemistry and Technology of Silicones, Walter Noll, Academic Press Inc, (Harcourt Brue Javanovich, Publishers, New York), 1968, pp 282-287 and 409-426.

### Surfactants

Classes of surfactants useful as emulsifiers for the compositions as disclosed herein include nonionic, anionic, cationic, amphoteric, synthetic emulsifying agents, and mixtures thereof. Many suitable nonionic and amphoteric surfactants are disclosed by U.S. Patent3,988,433; U.S. Patent 4,051,234, and many suitable nonionic surfactants are disclosed by U.S. Patent 3,959,458. Other emulsifiers optionally useful herein include natural emulsifying agents such as acacia, gelatin, lecithin and cholesterol; finely dispersed solids such as colloidal clays, bentonite, veegum (magnesium aluminum silicate; and synthetic emulsifying agents such as salts of fatty acids, sulfates such as sorbitan trioleate, sorbitan tristearate, sucrose distearate, propylene glycol monostearate, glycerol monostearate, propylene glycol monolaurate, sorbitan monostearate, sorbitan monolaurate, polyoxyethylene-4-lauryl ether, sodium lauryl sulfate, sulfonates such as dioctyl sosium sulfosuccinate, glyceryl esters, polyoxyethylene glycol esters and ethers, diethylene glycol monostearate, PEG 200 distearate, and sorbitan fatty acid esters, such as sorbitan monopalmitate, and their polyoxyethylene derivatives, polyoxyethylene glycol esters such as the monostearate, Polysorbate 80 (ethoxylated sorbitan monooleate) (supplied by Spectrum, etc.), and mixtures thereof.

### Water

The personal care composition may further comprise water, preferably from less than about 10% of water, less than about 8% water, less than about 5%, less than about 3%, all by weight of the composition. Alternatively, the personal composition can comprise from about 0.5% to about 10% water, alternatively from about 0.75% to about 8%, alternatively from about 1% to about 5% water, all by weight of the composition.

Preservatives, buffering agents, dyes, and flavoring agents, may be also included in some embodiments of the composition(s).

Examples of preservatives include, but are not limited to, sodium benzoate, benzoic acid, sorbic acid, and esters of p-hydroxybenzoic acid, parabens, in particular, propyl paraben, ethyl paraben, methyl paraben, benzalkonium chloride, ethylenediaminetetraacetic acid(EDTA), benzyl alcohol, potassium sorbate, or a mixture thereof. The composition may comprise from about 0.01% to about 1% preservative, from about 0.05% to about 0.5% preservative, from about 0.07% to about 0.3% preservative, and preferably from about 0.08% to about 0.15% preservative.

Examples of buffering agents include, but are not limited to, acetic acid, sodium acetate, citric acid, sodium citrate, monobasic sodium phosphate, dibasic sodium phosphate, sodium carbonate, sodium bicarbonate, succinic acid, sodium succinate, potassium dihydrogen phosphate, and phosphoric acid and help to establish and maintain a constant pH-value. The amount of buffering agents is determined and based on the pH-requirements to be met and can be adapted easily by the skilled person.

The present composition may further comprise any dye or color for increasing the optical acceptance of the consumer to be treated. Non-limiting examples of colors can include red, green, amber, orange, yellow, blue, pink, violet, turquoise, and combinations thereof. In one example, the composition is clear. The composition can also comprise a dye that provides the color. Non-limiting examples dyes that may be used in the present invention include FD&C blue #1, FD&C blue #2, D&C blue #4, D&C blue #9, FD&C green #3, D&C green #5, D&C green #6, D&C green #8, D&C orange #4, D&C orange #5, D&C orange #10, D&C orange #11, FD&C red #3, FD&C red #4, D&C red #6, D&C red #7, D&C red #17, D&C red #21, D&C red #22, D&C red #27, D&C red #28, D&C red #30, D&C red #31, D&C red #33, D&C red #34, D&C red #36, D&C red #39, FD&C red #40, D&C violet #2, FD&C yellow #5, FD&C yellow #6, D&C yellow #7, Ext. D&C yellow #7, D&C yellow #8, D&C yellow #10, D&C yellow #11, and combinations thereof. The composition may comprise from about 0.001% to about 0.1% dye, from about 0.002% to about 0.05% dye, or form about 0.003% to about 0.01% dye.

The composition as disclosed herein may further comprise fragrance materials that are acceptable to consumers. In one example, fragrance materials suitable for use herein can include cajeput oil, fennel oil, geranium oil, lemon oil, spearmint oil, eucalyptus oil, myrtle oil, oregano oil, pine oil, sarriette oil, thyme oil, cedar wood oil, cedar leaf oil, nutmeg and mixtures thereof. In addition or alternatively, the fragrance materials may comprise the chemical constituents of essential oils such as vanillin, ethyl vanillin, musk, methyl-dihydrojasmonate, anethol, catechol, camphor, camphene, ferulic acid, farnesol, hinokitiol, tropolone, menthol, levomenthol, methyl salicylate, carvacol, terpineol, verbenone, ratanhia extract, caryophyllene oxide, citronella acid, curcumin, nerolidol, turpentine, thymol, or mixtures thereof. These materials may be used at levels and ratios known to those skilled in the art of mixing fragrance materials for personal care compositions.

### Internal Administration

Alternatively, one or more comfreyn active(s), in particular comfreyn A and/or pharmaceutic compositions comprising them may also be administered internally by a systemic pharmaceutic composition. With a "systemic pharmaceutic composition" as used herein is meant a product which in the ordinary course of usage is used for purposes of systemic administration of therapeutic agents, which is applied by oral, parental, use of suppositories, or other internal and systemic application in a dosage form and dosage sufficient to achieve intended purposes, e.g. inhibition of inflammation, pain relief etc.. The compositions can include a variety of internally administered dosage forms. Non-limiting examples of dosage forms can include a liquid medication, particles suspended in a liquid formulation, a solid in a gelatin or foam, or a solid dose in the form of a tablet, capsule, powder, granules, pellets, microspheres, nanospheres, beads, or nonpareils, and combinations thereof.

Dosage forms that can be orally administered may be swallowed immediately, slowly dissolved in the mouth, or chewed. In some aspects the composition can be formulated as a powder, tablet, capsule, enteric-coated dosage form (e.g., for delivery to ileum/colon), or pill that can be administered to a subject by any suitable route. A lyophilized formulation can be mixed with a saline or other solution prior to administration.

In some aspects the pharmaceutic composition as disclosed herein may be allied enteric. An enteric coating can protect the actives of the composition, for example from the acidity of the stomach and provide delivery to the ileum and/or upper colon regions. Non-limiting examples of enteric coatings can include pH sensitive polymers (e.g., Eudragit^{®} FS30D), methyl acrylate-methacrylic acid copolymers, cellulose acetate succinate, hydroxy propyl methyl cellulose phthalate, hydroxy propyl methyl cellulose acetate succinate (e.g., hypromellose acetate succinate), polyvinyl acetate phthalate (PVAP), methyl methacrylate-methacrylic acid copolymers, shellac, cellulose acetate trimellitate, sodium alginate, zein, other polymers, fatty acids, waxes, shellac, plastics, and plant fibers. In some aspects the enteric coating is formed by a pH sensitive polymer, e. g using Eudragit^{®} FS30D.The composition inside the coating may be formulated similar to the topical composition as disclosed herein, with the prerequisite that only carrier materials that are intended and registered for internal administration are used, such as vegetable oils (e.g. peanut oil) or emulsifiers that are accepted for nutrition such as lecithine.

In addition, or alternatively, the pharmaceutic composition as disclosed herein may also be provided in form of a tablet. Composition for tablets are known by the skilled person and generally comprise fillers, such as starch, glucose, mannitol and/or sorbitol, binders, such as microcristalline cellulose, starch, starch paste, cellulose ether and/or gelatine, disintegrants, lubricants and/or an optional coating. The active agents may be dried and mixed directly with the tablet materials.

By "dose" as used herein is meant a volume of medication, such as liquid or solid medication, containing an amount of a drug active suitable for administration on a single occasion, according to sound medical practice. A dose can be orally administered. In one example, a dose can be about 30 mL, in another example about 25 mL, in another example about 20 mL, in another example about 15 mL, and in another example about 10 mL. In another example, a dose of liquid medication can be from about 10 mL to about 75 mL, in another example from about 15 mL to about 50 mL, in another example from about 25 mL to about 40 mL, and in another example from about 28 mL to about 35 mL. In another example, a dose can be half of a tablet or capsule, in another example one tablet or capsule, in another embodiment 1.5 tablets or capsules, and in another embodiment 2 tablets or capsules. In another example, a dose of solid medication can be from about 1 mg to about 100 mg, in another example from about 5 mg to about 50 mg, in another example from about 10 mg to about 40 mg, in another example from about 15 mg to about 30 mg, and in another example from about 50 mg to about 90 mg, in another example from about 55 mg to about 80 mg and in another example from about 60 mg to about 75 mg. The concentration of active ingredients can be adjusted to provide the proper doses of actives given the dose size. In one example, the dose is intended to be administered every 4 hours, in another example every 6 hours, in another example every 8 hours, and in another example every 12 hours.

The active agents disclosed herein, including but not limited to one or more comfreyn(s), in particular comfreyn A, the combination of comfreyn A with caffeic acid ethyl ester as well as further actives mentioned herein and the pharmaceutic compositions comprising said actives can be used, in particular, in the therapy of pain, inflammation, swellings, restricted movements, contusions, strains, sprains, joint arthrosis, back pain, muscle complaints and/or joint complaints. Without wishing to being bound by a theory the therapeutic effect is achieved in particular via the NF-κB route by inhibition of E-selectine transcription.

Thus, the present invention also refers to a method of inhibiting and/or treating inflammation by applying topically and/or administering internally a safe and effective amount of one or more comfreyn(s), in particular comfreyn A or a combination of a safe and effective amount of comfreyn A and a safe and effective amount of caffeic acid ethyl ester and/or further additional active agents as disclosed herein.

The present invention further refers to a method of inhibiting and/or treating inflammation by blocking at least partially the activation of the NF-κB route after activation, e.g. with interleukin 1β (IL-1β), by applying topically and/or administering internally a safe and effective amount of one or more comfreyn(s), in particular comfreyn A or a combination of a safe and effective amount of comfreyn A and a safe and effective amount of caffeic acid ethyl ester and/or further additional active agents as disclosed herein.

The pharmaceutic composition may be applied topically and/or may be administered internally. Topical application requires application of the pharmaceutic composition in a sufficient amount to achieve an effect to the area in need of treatment, wherein the composition remains there for a sufficient period of time to achieve the effect.

The present invention further relates to a kit comprising a pharmaceutic composition as disclosed herein comprising the one or more arylnaphthalene lignan(s), in particular comfreyn A or the comfreyn A plus caffeic acid ethyl ester in a safe and effective amount and instructions to use. The using instruction will describe some example application scenarios as described herein, but the optimal amount, duration and frequency of application will depend on the desired effect, the severity of any condition being treated, the health and age of the user and like considerations.

Further preferred embodiments are based on the dependent patent claims. Features of the composition claims can be combined correspondingly with the use claims here and vice versa in any desired combination. In the following the present invention will be described in more detail.

### EXAMPLES

### General experimental procedures

IR measurements were carried out on a Bruker IFS-48 spectrometer. UV spectra were obtained on a Beckman DU 670 spectrometer. NMR experiments were acquired in methanol-d4 (99.95%, Sigma-Aldrich) on a Bruker DRX-600 spectrometer (Bruker BioSpin GmBH, Rheinstetten, Germany) equipped with a Bruker 5 mm TCI CryoProbe at 300 K. Data processing was carried out with Topspin 3.2 software. The ROESY spectra were acquired with tmix = 400 ms. Size exclusion chromatography was performed over Sephadex LH-20 (GE Healthcare, Sigma Aldrich, Milan, Italy).

### Compound Isolation

A PA-depleted hydroalcoholic (20% EtOH w/w) liquid extract of comfrey roots (DER 1:2) was obtained from Merck KGaA &Co Werk Spittal, Austria. To remove the mucilage, the ethanol content of 500g of liquid extract was evaporated and the aqueous phase was partitionated with ethylacetate (1:1), obtaining 50g of ethyl acetate extract. 3g of ethyl acetate extract were fractionated on a Sephadex LH-20 (GE Healthcare, Sigma Aldrich, Milan, Italy) column (100 × 5 cm), using methanol as mobile phase, affording 80 fractions (8 mL), monitored by LC-ESI-HR-MS analysis.

### LC-ESI/LTQOrbitrap/MS analysis

The LC-MSⁿ guided fractionation of a mucilage- and PA-depleted extract of comfrey root showing inhibition of interleukin-1β-induced expression of E-selectin in HUVEC cells at a concentration of 20 µg/mL was analyzed by LC-ESI/HR/MS using the "data dependent scan" mode in which precursor ions corresponding to the most intense peaks are selected. The procedure was performed as shown in the following:
The secondary metabolite profile of the hydroalcoholic extract of comfrey roots was obtained by using a HPLC method coupled to a hybrid mass spectrometer, which combines the linear trap quadrupole (LTQ) and Orbitrap mass analyzer. All experiments were carried out using a Thermo scientific liquid chromatography system constituted of a quaternary Accela 600 pump and an Accela autosampler, connected to a linear Trap-Orbitap hybrid mass spectrometer (LTQ-Orbitrap XL, Thermo Fisher Scientific, Bremen, Germany) with electrospray ionization (ESI). Separation was performed on a Kinetex EVO C18 5 µm (150 mm × 2.10 mm) column (Phenomenex Aschaffenburg, Germany) using a flow rate of 0.2 mL/min and a mobile phase consisting of a combination of A (0.1% formic acid in water, v/v) and B (0.1% formic acid in acetonitrile, v/v). A linear gradient program starting with isocratic at 5% B in 5 min, then from 5 to 23% B in 5 min, held at 23% B for 5 min, from 23 to 55% B in 7 min, from 55 to 95% B in 5 min, held at 95% B for 5 min was used. The mass spectrometer operated in negative ion mode. ESI source parameters were as follows: capillary voltage -48V; tube lens voltage -176.47; capillary temperature 280°C; sheath and auxiliary gas flow (N2), 15 (arbitrary units) and 5 (arbitrary units), sweep gas 0 spray voltage 5 kV. The mass range was from 120 to 1600 m/z with a resolution of 30000. For fragmentation study, a data dependent scan was performed, selecting precursor ions corresponding to the most intense peaks in LC-MS analysis. Xcalibur software version 2.1 was used for instrument control, data acquisition and data analysis.

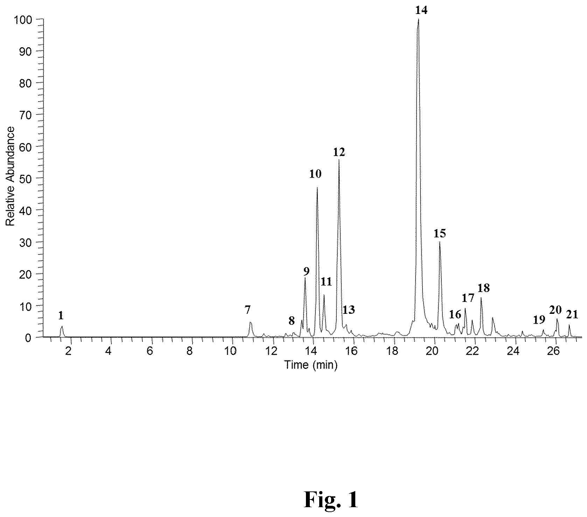

A preliminary metabolite profiling showed 20 main compounds (Fig. 1) corresponding to different structural classes (Fig. 2). Some of these metabolites were identified by comparison of their accurate masses, characteristic fragmentation patterns, and retention times to those of authentic standards. On the basis of the above approach, the occurrence of allantoin (1), protocatechuic acid (4), protocatechuic aldehyde (5), p-hydroxy benzoic acid (6), caffeic acid (7) as well as its ethyl ester derivative (16), rosmarinic acid (12) along with the fatty acids linolenic (18), linoleic (19) and hydroxypalmitic (20) were identified. The remaining compounds (2, 3, 8-11, 13-15 and 17) could not be unambiguously assigned by HR-ESI-MS either because their accurate masses did not match to any of the compounds reported from *S. officinale* in the literature or because their preliminary NMR data did not support compounds reported in *S. officinale* with the same molecular weights.

### Isolation procedure of comfreyn A (compound 15)

To fully characterize compounds 2-3, 8-11, and 13-17, the mucilage fraction of the extract was first removed by partitioning the liquid extract with ethyl acetate. Then, the organic phase was subjected to size exclusion chromatography on a Sephadex LH-20 column and further purified by semipreparative HPLC-UV. Indeed, the obtained dried fractions were diluted in acetonitrile (at a concentration of 10 mg dry residue /100 µL), and submitted to semipreparative HPLC UV separations by using a Phenomenex C18 Synergi-Hydro-RP (250 mm × 10 mm, 10 µm) column on an Agilent 1260 Infinity system (Agilent Technologies, Palo Alto, CA, USA), equipped with a binary pump (G-1312C), and an UV detector (G-1314B). The mobile phase consisted of solvent A (H2O + 0.1 % formic acid) and solvent B (CH3CN + 0.1% formic acid). The analyses were performed at room temperature without thermostating. The peaks collected at HPLC-UV have been evaporated to dryness in vacuum and also freeze dried before NMR analysis. The compound structures were fully characterized by extensive ID- and 2D-NMR experiments as well as MS/MS analysis.

Fraction 23-26 (10 mg) corresponded to compound 12, namely rosmarinic acid.

Fraction 40-42 (10 mg) corresponded to compound 14, which was identified as globoidnan A (C₂₆H₂₀O₁₀).

Fractions 19-22 (51.6 mg) were purified using a linear gradient program at a flow rate of 2 mL/min at a wavelength of 254 nm (from 0 to 20% B in 15 min; from 20 to 40% B in 10 min; from 40 to 60% B in 10 min; from 60 to 100% B in 11 min, and then followed by 6 min at 100% B), to afford compounds 2 (1.2 mg, Rt= 10.3 min), 3 (1.3 mg, Rt= 13.5 min), 6 (1.5 mg, Rt= 11.2 min, p-hydroxy benzoic acid) and 9 (2.2 mg, Rt= 27.3). NMR analysis confirmed that compound 2 as *p*-hydroxy benzoic acid glucoside and compound 3 as 5-hydroxymethyl-2-furfural, respectively. Compound 9 showed a majorion peak at *m*/*z* 367.1384 [M-H]⁻ supporting the molecular formula C₁₈H₂₄O₈. Its MS/MS spectrum showed a fragment ion at *m*/*z* 205.09 [M-H- 162]⁻ suggesting the presence of a hexose unit. NMR data of 9 allowed to deduce its structure as the glucosylated and prenylated phenolic acid malaxinic acid which is described fist time to be present in *S. officinale* herein.

Fractions 27-39 (42.3 mg), Fractions 43-54 (7.1 mg) and Fractions 55-80 (22.2 mg) were purified in the same elution conditions at a flow rate of 2 mL/min and at a wavelength of 280 nm: (0-5 min held at 5% B, from 5 to 20% B in 15 min; from 20 to 50% B in 20 min, from 50 to 100% B in 10 min and held at 100% B for 5 min).

In these conditions, compounds 4 (1.1 mg protocatechuic acid, Rt= 22.60 min), 5 (1.2 mg protocatechuic aldehyde, Rt=27.9min), 7 (1.5 mg caffeic acid, Rt=29.4min), 8 (1.3 mg, Rt=31.7 min), 11 (1.6 mg, Rt=34.3), 13 (1.7 mg, Rt=35.1 min), and 10 (4.4 mg, Rt=35.3) were isolated from Fractions 27-39.

The HR-ESI-MS spectra of 8, 11, and 14 supported molecular formulae of C₂₇H₂₂O₁₂, C₃₆H₃₀O₁₆, and C₂₆H₂₀O₁₀, respectively. Analysis of their NMR and MS/MS data allowed to identify compound 14 as globoidnan A and compound 8 as globoidnan B, respectively. Compound 11 was established as (+)-rabdosiin, wherein these lignans are first time described in roots *of S. officinale.* The molecular formula of compound 13 was established as C₉H₁₀O₅ and the structural elucidation afforded by NMR analysis allowed to identify compound 13 as hydroxy caffeic acid. Although, the MS/MS fragmentation patterns suggested a similar structural framework as that of globoidnan A for compound 10, the interpretation of the NMR data confirmed this hypothesis establishing the structure of 10 as 3-carboxy-6,7-dihydroxy-1-(3',4'-dihydroxyphenyl)-naphthalene.

Compound 15 (1.6 mg Rt= 41.10) was isolated from fractions 43-54.

Compound 16 (1.2 mg, Rt=42.2), was identified as caffeic acid ethyl ester and 17 (1.0 mg, Rt=43.3) were isolated from fractions 55-80. Although, the MS/MS fragmentation patterns suggested a similar structural framework as that of globoidnan A for compound 17, the interpretation of the NMR data established the structure of compound 17 as the ethyl ester derivative of compound 10, ternifoliuslignan D.

Moreover, 3g of raw material were extracted with 50 mL of methanol to obtain the methanol extract in order to prove the natural occurrence of the ethyl esters 15-17 in comfrey by LC-MS analysis. Therewith, it was shown that compounds 15, 16 and 17 are not artefacts due to the use of ethanol as extraction solvent but occur naturally.

### Identification of compound 15; named comfreyn A:

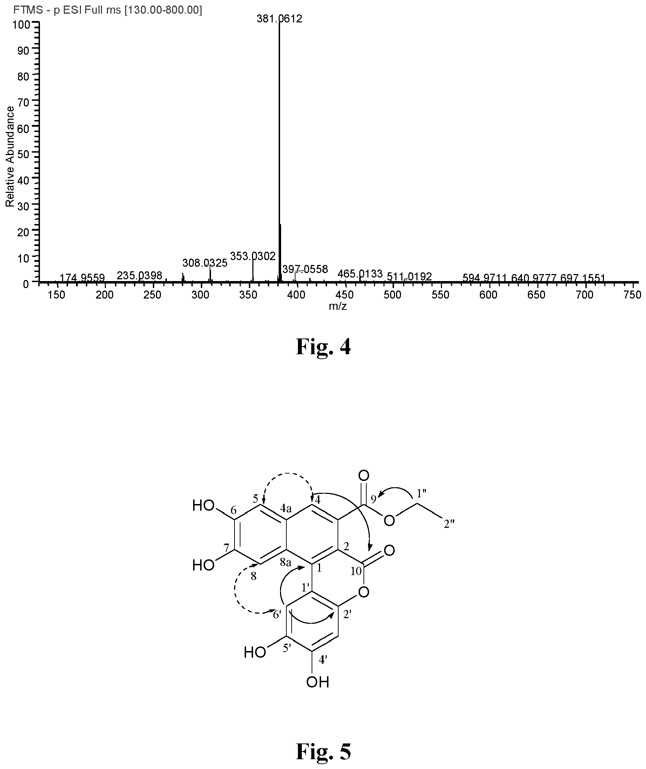

Comfreyn A (compound 15) appeared as an amorphous yellow solid. The HR-ESI-MS spectrum of compound 15 *(m*/*z* 381.0601 [M-H]⁻, calculated for C₂₀H₁₃O₈, 381.0610) supported a molecular formula of C₂₀H₁₄O₈ (Fig. 4). The ESI/MS/MS spectra of compound 15 displayed an intense ion at *m*/*z* 353.02 ([M-H-28]⁻) suggesting the presence of an ethyl group. MS of the ion peak at *m*/*z* 353.02 displayed fragment ions at *m*/*z* 309.10 ([M-H-44]⁻) and 265.10 ([M-H-44-44]⁻) corresponding to the loss of two CO₂ molecules. 1H and 13C NMR (methanol-d4, 600 MHz and 150 MHz) data are shown in Table 1 below; ESI-HR-MS m/z 381.0601 [M-H]- (calcd for C20H13O8, 381.0610).

**Table 1: 13C (150 MHz) and 1H NMR (600 MHz) data of compound 15.**

| | δC | δH (J in Hz) | HMBC (H→C) correlations |
|---|---|---|---|
| 1 | 136.2 | - | |
| 2 | 113.8 | - | |
| 3 | 130.5 | - | |
| 4 | 126.6 | 7.67, s | C-1, C-2, C-4, C-4a,C-8a, C-9, C-10 |
| 4a | 133.6 | - | |
| 5 | 112.4 | 7.34, s | C-4, C-8a,C-6, C-7 |
| 6 | 151.3 | - | |
| 7 | 150.2 | - | |
| 8 | 111.5 | 8.30, s | C-1, C-4a,C-6, C-7 |
| 8a | 124.5 | - | |
| 9 | 172.1 | | |
| 10 | 162.4 | - | |
| 1' | 111.5 | - | |
| 2' | 144.0 | - | |
| 3' | 104.5 | 6.91, s | C-1, C-1', C-2',C-4', C-5' |
| 4' | 149.6 | - | |
| 5' | 147.1 | - | |
| 6' | 113.8 | 8.02, s | C-1, C-1, C-2',C-4', C-5' |
| 1" | 62.8 | 4.43, q (7.0) | C-9, C-2" |
| 2" | 14.2 | 1.41, t (7.0) | C-1" |

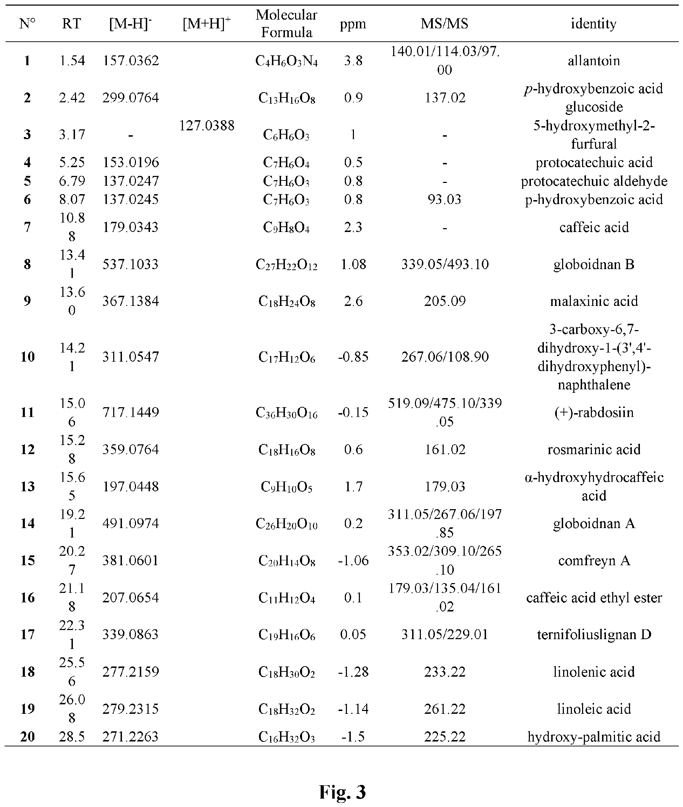

The analysis of the ¹³C and HSQC NMR data for compound 15 suggested a highly unsaturated molecule bearing 16 aromatic carbons whereas its 1H NMR spectrum displayed five aromatic singlets at δ 8.30 (H-8), 8.02 (H-6'), 7.67 (H-4), 7.34 (H-5), 6.91 (H-3'). Additionally, 1H NMR of compound 15 showed characteristic signals of an ethoxy group at δ 4.43 (2H, q, J = 7.0 Hz) and 1.41 (3H, t, J = 7.0 Hz). Moreover, a detailed analysis of the HMBC spectrum confirmed the presence of two main fragments (Fig. 5). In particular, key long-range correlations between the aromatic proton H-4 and the carbon resonances at δ 172.2 (C-9), 130.5 (C-3), 113.4 (C-2), 133.7 (C-4a), 112.4 (C-5), and 124.5 (C-8a), H-5 and the carbon resonances at δ 133.7, 151.3 (C-6), 150.3 (C-7), and 124.5, and H-8 and the carbon resonances at δ 136.1 (C-1), 151.3, and 133.7 established the occurrence of a 1,2-disubstituted 6,7-dihydroxy-naphtalene-3-carboxylic acid. The structure of the second fragment was established as 1-substituted benzene-2,4,5-triol based on HMBC correlations from protons H-3' and H-6' to the carbon resonances at δ 111.5 (C-1'), 143.8 (C-2'), 149.6 (C-4'), and 147.1 (C-5'). In turn, connectivity between these two fragments was deduced from the HMBC correlation from H-6' to C-1. In addition, a HMBC correlation from the equivalent methylene at *δ* 4.43 (H-1") to the carbonyl at δ 172.2 linked the ethoxy residue to the naphthalene moiety. Inspection of this partial structure revealed that it contained 13 of the required 14 degrees of unsaturation and only one of the two carbonyls was assigned. Consequently, the phenol ring and the 6,7-dihydroxy-naphtalene-3-carboxylic acid residue had to connect via formation of a lactone ring to satisfy the unsaturation index and molecular formula. HMBC experiments with long-range delays ranging from 2.75 to 10 Hz were performed. The HMBC spectrum, optimized for the ⁿ*J*(C,H) coupling of 5 Hz, corresponding to a delay time Δ2=100 ms, showed four-bonds long range correlations and stronger two-bond long range correlations. Finally, the structure of compound 15 was assembled as follows. A ⁴*J*_{CH} HMBC correlation from H-4 to the carbon resonance at δ 162.5 (C-10) allowed to deduce the linkage of the carbonyl group to C-2, whereas a ROE correlation between H-6' and H-8 along with a ⁴*J*_{CH} HMBC correlation from H-6' to C-2 indicated the esterification site at C-2 (Fig. 3). Therefore, comfreyn A (15) was established as an arylnaphtalene lignan bearing an unusual δ-lactone ring, corresponding to a coumarin skeleton.

### Pharmaceutic activity of Comfreyn A

J. Seigner et al. (Frontiers in Pharmacology 10.3389/fphar.2019.00289) reported recently that interleukin-1β (IL-1β) induced E-selectin mRNA levels were inhibited by a comfrey extract by approximately 70% at the starting concentration of 20 µg/ml. E-selectin is a NP-κB-dependent target gene and IL-1β is a well-described pro-inflammatory mediator that exerts an important role in the regulation of immune and inflammatory responses, in particular those involving sterile insults such as trauma and blunt injuries. The tested extract was shown to comprise allantoin (1), protocatechuic acid (5), p-hydroxybenzoic acid (6), caffeic acid (7), rosmarinic acid (12) and globoidnan A (14). Thus, the anti-inflammatory activity of individual compounds 1, 5, 12 and 14, was tested using IL-1-stimulated HUVEC cells and analyzed by real time PCR determining the fold change in mRNA expression of E-selectin. These were compared to the inhibitory activity of the new arylnaphthalene lignan comfreyn A (15) and globoidnan B (8), malaxinic acid (9) and caffeic acid ethyl ester (16), the latter three identified first time in *S. officinale* as disclosed herein.

Therefore, primary HUVEC cells were isolated from umbilical cords as described by M. Hoeth et al., Guidance Molecules in Human Endothelial Cells, Plos One 7, 2012 and maintained in M199 medium (Lonza) supplemented with 20% FCS (Sigma), 2 mM L-glutamine (Sigma), penicillin (100 units/mL), streptomycin (100 mg/mL), 5 units/mL heparin, and 25 mg/mL ECGS (Promocell), and were used up to passage 5. HUVEC cell were preincubated with various concentration of purified compounds (1, 5, 8, 9, 12, 14, 15 and 16) for 30 minutes following stimulation with 5 ng/mL IL-1β (R&D Systems) for 90 min. Cells were then analyzed for the change in mRNA expression of E-Selectin (SELE). Therefore, total RNA was isolated using the PeqGold Total RNA Isolation Kit (VWR International) according to the manufacturer's instructions. 1 µg RNA was reverse transcribed using random hexamers (Fermentas) and murine leukemia virus reverse transcriptase (Thermo Scientific Fisher). Primers were designed using the software "Primer3". Following primer sequences were used for qPCR: glyceraldehyde 3-phosphate dehydrogenase (GAPDH) forward, 5'-AGAAGGCTGGGGCTCATTT-3' and reverse, 5'-CTAAGCAGTTGGTGGTGCAG-3'; E-Selectin (SELE) forward, 5'-CCTGTGAAGCTCCCACTGA-3', and reverse 5'-GGCTTTTGGTAGCTTCCATCT-3'. Real-time PCR was done with the SsoAdvanced Universal SYBR Green Supermix (BioRad) using the StepOnePlus instrument (Applied Biosystems), and relative mRNA expression normalized to GAPDH. Fold changes in mRNA expression were calculated according to the 2-ΔΔCT method. Results are presented as mean fold induction of averaged Ct-values of triplicates and are shown in Table 2:

**Table 2: Inhibitory effect of 1, 5, 8, 9, 12, 14, 15 and 16 on IL-1β induced E-selectin expression.**

| Compound | EC [µM] | Max. Inhibition (%) |
|---|---|---|
| 1 | - | - |
| 5 | 120 | 50 |
| 8 | - | - |
| 9 | 108 | 60 |
| 12 | - | - |
| 14 | 40 | 35 |
| 15 | 50 | 52 |
| 16 | 64 | 70 |

| | | |
|---|---|---|
| Effective concentration (EC) is based at least on three independent experiments. | | |

Surprisingly, it was shown that allantoin (compound 1) and rosmarinic acid (compound 12) do not show any inhibitor activity against E-selectin expression. A small effect of 35% inhibition could be achieved with globoidnan A (compound 14) at an effective concentration of 40µM, wherein the formerly know globoidnan B (compound 8) did not show any inhibitory activity. Comfreyn A (compound 15) was found to be more effective in E-selectin inhibition with a 50% inhibition at an EC of 50µM. Caffeic acid ethyl ester (compound 16) represents the most active inhibitor inhibiting E-selectin expression by 70% at a concentration of 64 µM. The already known protocatechuic acid (compound 5) as well as the newly identified malaxinic acid (compound 9) also showed an inhibitory effect above 50%, but only at higher effective concentrations. Comfreyn A (15) and caffeic acid ethyl ester (16) compared to the latter two compounds are effective at half concentration.

### EXAMPLE COMPOSITIONS

In the following academic example compositions are specified, which are intended to be exemplary only and shall not limit the scope of the present invention.

| | Cream | W/O ointment - Oleaginous base | Vanishing cream |
|---|---|---|---|
| Ingredients | %w/w | %w/w | %w/w |
| Comfrey root extract | 2.5 - 5 | 2.5 - 5 | 2.5 - 5 |
| Olive oil | 5 - 15 | 15 - 60 | --- |
| Cetostearyl Alcohol | 4 - 10 | 4 - 10 | --- |
| Stearic acid | --- | --- | 5 -20 |
| Medium chain triglyceride of C8/C12 acid | --- | --- | 5 - 15 |
| Glyceryl Monostearate | 2 - 6 | 2 - 6 | 1 - 5 |
| Sodium Lauryl Sulphate | 0.5 - 2.5 | 0.5 - 2.5 | 0.5 - 2.5 |
| Potassium hydroxide (KOH) | --- | --- | 0.2 - 1 |
| Disodium EDTA | --- | --- | 0.05 - 1 |
| Glycerol | 3 - 10 | 3 - 10 | 3 - 10 |
| Vanillyl Butyl Ether / Levo Menthol | 0.1 - 1 / 2-5 | 0.1 - 1 / 2 - 5 | 0.1 - 1 / 2 - 5 |
| Winter green oil | 0.5 - 2 | 0.5 - 2 | 0.5 - 2 |
| Alpha tocopherol / Oxynex | 0.1 - 1 | 0.1 - 1 | 0.1 - 1 |
| Phenoxyethanol | 0.1 - 1 | 0.1 - 1 | 0.1 - 1 |
| Water | Qs up to 100 | Qs up to 100 | Qs up to 100 |

| Ingredients | Hydrogel w Carbomer | Hydrogel w Poloxamer | Emulgel | Ointment water soluble base | Liposomal formulation |
|---|---|---|---|---|---|
| | %w/w | %w/w | %w/w | %w/w | %w/w |
| Comfrey root extract | 2.5 - 5 | 2.5 - 5 | 2.5 - 5 | 2.5 - 5 | 2.5 - 5 |
| Carbomer | 0.5 - 2 | --- | 0.5 - 2 | --- | --- |
| Poloxamer | --- | 18 - 25 | 18 - 25 | --- | --- |
| Medium chain triglyceride of C8/C12 acid | --- | --- | 5 - 20 | --- | --- |
| PEG-40 Hydrogenated Castor Oil | --- | --- | 2 - 10 | --- | --- |
| PEG-400 | --- | --- | --- | 50 - 60 | --- |
| PEG 3350 | --- | --- | --- | 30 - 40 | --- |
| Lecithin | --- | --- | --- | --- | 0.2 - 20 |
| Glycerol | 3 - 10 | 3 - 10 | --- | --- | --- |
| Cholesterol | --- | --- | --- | --- | 0.2 - 20 |
| Triethanol amine | Qs to neutral pH | --- | Qs to neutral pH | --- | --- |
| Alpha tocopherol / Oxynex | --- | --- | 0.1 - 1 | --- | --- |
| Phenoxyethanol | 0.1 - 1 | 0.1 - 1 | 0.1 - 1 | 0.1 - 1 | --- |
| Sodium deoxy cholate | --- | --- | --- | --- | 0.2 - 5 |
| Water | Qs 100 | Qs 100 | Qs 100 | Qs 100 | --- |
| Water / buffer pH 4.5 to 5.5 | --- | --- | --- | --- | 10 - 20 |
| Cream base / gel base | --- | --- | --- | --- | Qs 100 |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. One or more arylnaphthalene lignan(s) having the general structure as shown in Formula I 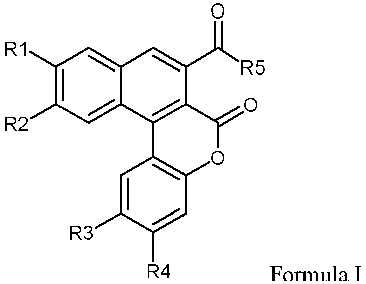 wherein each R1, R2, R3, and R4 are independently selected from hydrogen, hydroxy, methyl, methoxy, ethoxy, propoxy, butoxy or pentoxy; and
wherein R5 is selected from hydroxy, methoxy, ethoxy, propoxy, butoxy, pentoxy, prenoxy, isoprenoxy, feruloyl, caffeoyl, or coumaroyl residues.

2. The arylnaphthalene lignan(s) according to claim 1, wherein R1, R2, R3 and R4 each are hydroxy and R5 is ethoxy.

3. A pharmaceutical composition comprising
a) an active agent comprising one or more of the arylnaphthalene lignan(s) according to anyone of Claims 1 or 2; and
b) a pharmaceutically acceptable carrier.

4. The pharmaceutical composition according to claim 3 further comprising an additional active agent, wherein the additional active agent is preferably a pain reliver, an active agent with anti-inflammatory activity, an activity enhancer or a combination thereof, more preferred wherein the additional active agent is a natural pain reliever, more preferred wherein the additional active agent is a natural pain reliver extracted from *Symphytum officinale* L.

5. The pharmaceutical composition according to claim 4 wherein the additional active agent comprises caffeic acid ethyl ester, malaxinic acid, globoidnan A, protocatechuic aldehyde, a *Symphytum officinale* L. root extract or a combination thereof, preferably wherein the additional active agent comprises caffeic acid ethyl ester, malaxinic acid, globoidnan A or a combination thereof.

6. The pharmaceutical composition according to claims 4 or 5, wherein the additional active agent further comprises allantoin, *p*-hydroxy benzoic acid glucoside, 5-hydroxymethyl-2-furfural, protocatechuic acid, *p*-hydroxy benzoic acid, caffeic acid, globoidnan B, 3-carboxy-6,7-dihydroxy-1-(3',4'-dihydroxyphenyl)-naphthalene, (+)-rabdosiin, rosmarinic acid, alpha-hydroxyhydro caffeic acid, ternifoliuslignan D, linolenic acid, linoleic acid, hydroxypalmitic acid or a combination thereof, preferably comprises globoidnan B, ternifoliuslignan D, 3-carboxy-6,7-dihydroxy-1-(3',4'-dihydroxyphenyl)-naphthalene, rabdosiin, and/or a combination thereof.

7. The pharmaceutical composition according to anyone of claims 3 to 6, wherein the composition comprises the one or more arylnaphthalene lignan(s) active agent in a pharmaceutically safe and effective amount, preferably in an amount to provide a local concentration from 30µM to 65µM, more preferred in an amount to provide a local concentration from 40µM to 60µM, more preferred in an amount to provide a local concentration from 45µM to about 55µM, more preferred in an amount to provide a local concentration from 48µM to 52µM active agent at the location in need thereof, more preferred wherein the composition comprises the one or more arylnaphthalene lignan(s) active agent in an amount from 1µg/g of composition to 10µg/g of composition, more preferred in an amount from 2µg/g of composition to 7.5µg/g of composition, and even more preferred in an amount from 3µg/g of composition to 5µg/g of composition.

8. The pharmaceutical composition according to anyone of claims 5 to 7, wherein the composition comprises caffeic acid ethyl ester in a safe and effective amount, preferably in an amount to provide a local concentration from 50µM to 80µM, more preferred in an amount to provide a local concentration from 55µM to 75 µM, more preferred in an amount to provide a local concentration from 60µM to about 70µM, more preferred in an amount to provide a local concentration from 62µM to 66µM caffeic acid ethyl ester at the location in need thereof, more preferred wherein the composition comprises the caffeic acid ethyl ester more in an amount from 1µg/g of composition to 15µg/g of composition, more preferred in an amount from 2.5µg/g of composition to 10µg/g of composition, and even more preferred in an amount from 5µg/g of composition to 7.5µg/g of composition.

9. The pharmaceutical composition according to anyone of claims 3 to 8, wherein the composition inhibits E-selectine transcription after activation with Interleukin 1β by at least 30%, preferably by at least 40%, more preferred by at least 45%, more preferred by at least 48%, even more preferred by at least 50%.

10. The pharmaceutical composition according to anyone of claims 3 to 9, wherein the composition is a topical composition or a composition for internal administration, preferably wherein the topical composition is in the form of an ointment, a salve, a paste, a viscous liquid, a lotion, a gel, a cream, a mousse, a foam, a coating or any other adhesive product, applied to a strip or a dissolvable strip or a combination thereof and/or wherein the composition for internal administration is preferably in form of a capsule, a tablet, a lozenge,a chewable tablet, a chewing gum, a syrup, a liquid, a mousse or a combination thereof.

11. The pharmaceutical composition according to claim 10, wherein the pharmaceutically acceptable carrier is a water-in-oil emulsion comprising at least one carrier base material, a surfactant and water, preferably wherein the carrier base material is selected from non-toxic oil, non-toxic edible oils, saturated or unsaturated fatty alcohols, aliphatic hydrocarbons, long chain triglycerides, fatty esters, and mixtures thereof, more preferably wherein the carrier base material is peanut oil.

12. The pharmaceutical composition according to anyone of claims 3 to 11, wherein the composition further comprises at least one of preservatives, dyes, fragrances, stabilizers, buffering agents or a combination thereof.

13. The pharmaceutical composition according to anyone of claims 3 to 12, wherein the composition comprises at least the arylnaphthalene lignan comfreyn A according to claim 3, caffeic acid ethyl ester, malaxinic acid and globoidnan A in a pharmaceutically acceptable carrier comprising at least one non-toxic edible oil, at least one emulsifier, at least one buffering agent, water and preservatives, preferably wherein the composition comprises comfreyn A in an amount to provide a local concentration about 50µM comfreyn A at the location in need thereof and caffeic acid ethyl ester in an amount to provide a local concentration about 65µM caffeic acid ethyl ester at the location in need thereof, more preferably wherein the composition comprises comfreyn A in an amount from 3µg/g composition to 5µg/g composition and caffeic acid ethyl ester in an amount from 5µg/g composition to 7.5µg/g composition, in a pharmaceutically carrier comprising preferably at least peanut oil, at least one emulsifier, at least one buffering agent, water, fragrances and preservatives.

14. The one or more arylnaphthalene lignan(s) according to anyone of claims 1 or 2 and the pharmaceutical composition according to anyone of claims 3 to 13 for use in inhibition and/or treatment of inflammation, preferably for the treatment of broken bones, tendon damages, painful joints and muscles, acute upper and lower back pain, gonarthrosis, blunt injuries, for reduction of swelling of joint distortions, for improvement of joint mobility in osteoarthritic joints and/or combinations thereof.

15. Use of the arylnaphthalene lignan comfreyn A according to Claim 2 as marker substance for quantitative analysis of the active substance in products using and/or extracted from comfrey root.

## Patentansprüche

1. Ein oder mehrere Arylnaphthalenlignane mit der allgemeinen Struktur, wie in Formel I gezeigt 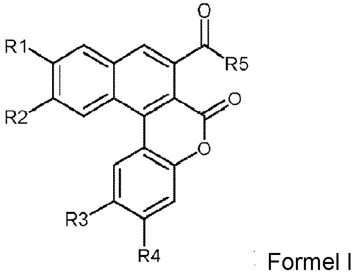 wobei jedes R1, R2, R3 und R4 unabhängig ausgewählt ist aus Wasserstoff, Hydroxy, Methyl, Methoxy, Ethoxy, Propoxy, Butoxy oder Pentoxy; und
wobei R5 ausgewählt ist aus Hydroxy, Methoxy, Ethoxy, Propoxy, Butoxy, Pentoxy, Prenoxy, Isoprenoxy, Feruloyl, Caffeoyl oder Cumarylresten.

2. Arylnaphthalenlignan(e) nach Anspruch 1, wobei R1, R2, R3 und R4 jeweils Hydroxy sind und R5 Ethoxy ist.

3. Arzneimittelzusammensetzung, umfassend
a) einen Wirkstoff, der eines oder mehrere des Arylnaphthalenlignans bzw. der Arylnaphthalenlignane nach einem der Ansprüche 1 oder 2 umfasst; und
b) einen pharmazeutisch verträglichen Träger.

4. Arzneimittelzusammensetzung nach Anspruch 3, ferner umfassend einen zusätzlichen Wirkstoff, wobei der zusätzliche Wirkstoff vorzugsweise ein Schmerzmittel, ein Wirkstoff mit entzündungshemmender Aktivität, ein Aktivitätsverstärker oder eine Kombination davon ist, wobei mehr bevorzugt der zusätzliche Wirkstoff ein natürliches Schmerzmittel ist, wobei mehr bevorzugt der zusätzliche Wirkstoff ein natürliches Schmerzmittel ist, das aus *Symphytum officinale* L. extrahiert wurde.

5. Arzneimittelzusammensetzung nach Anspruch 4, wobei der zusätzliche Wirkstoff Kaffeesäureethylester, Malaxinsäure, Globoidnan A, Protocatechualdehyd, einen *Symphytum-officinalis-*L.*-*Wurzelextrakt oder eine Kombination davon umfasst, wobei vorzugsweise der zusätzliche Wirkstoff Kaffeesäureethylester, Malaxinsäure, Globoidnan A oder eine Kombination davon umfasst.

6. Arzneimittelzusammensetzung nach den Ansprüchen 4 oder 5, wobei der zusätzliche Wirkstoff ferner Allantoin, *p*-Hydroxybenzoesäureglucosid, 5-Hydroxymethyl-2-furfural, Protocatechusäure, p-Hydroxybenzoesäure, Kaffeesäure, Globoidnan B, 3-Carboxy-6,7-dihydroxy-1-(3',4'-dihydroxyphenyl)-naphthalen, (+)-Rabdosiin, Rosmarinsäure, alpha-Hydroxyhydrokaffeesäure, Ternifoliuslignan D, Linolensäure, Linolsäure, Hydroxypalmitinsäure oder eine Kombination davon umfasst, vorzugsweise Globoidnan B, Ternifoliuslignan D, 3-Carboxy-6,7-dihydroxy-1-(3',4'-dihydroxyphenyl)-naphthalen, Rabdosiin und/oder eine Kombination davon umfasst.

7. Arzneimittelzusammensetzung nach einem der Ansprüche 3 bis 6, wobei die Zusammensetzung das eine oder die mehreren Arylnaphthalenlignan(e) in einer pharmazeutisch sicheren und wirksamen Menge umfasst, vorzugsweise in einer Menge, um eine lokale Konzentration von 30 µM bis 65 µM bereitzustellen, mehr bevorzugt in einer Menge, um eine lokale Konzentration von 40 µM bis 60 µM bereitzustellen, mehr bevorzugt in einer Menge, um eine lokale Konzentration von 45 µM bis etwa 55 µM bereitzustellen, mehr bevorzugt in einer Menge, um eine lokale Konzentration von 48 µM bis 52 µM Wirkstoff an der Stelle bereitzustellen, an der dieser benötigt wird, wobei mehr bevorzugt die Zusammensetzung den einen oder die mehreren Arylnaphthalenlignan-Wirkstoffe in einer Menge von 1 µg/g der Zusammensetzung bis 10 µg/g der Zusammensetzung, mehr bevorzugt in einer Menge von 2 µg/g der Zusammensetzung bis 7,5 µg/g der Zusammensetzung und noch mehr bevorzugt in einer Menge von 3 µg/g der Zusammensetzung bis 5 µg/g der Zusammensetzung umfasst.

8. Arzneimittelzusammensetzung nach einem der Ansprüche 5 bis 7, wobei die Zusammensetzung Kaffeesäureethylester in einer sicheren und wirksamen Menge umfasst, vorzugsweise in einer Menge, um eine lokale Konzentration von 50 µM bis 80 µM bereitzustellen, mehr bevorzugt in einer Menge, um eine lokale Konzentration von 55 µM bis 75 µM bereitzustellen, mehr bevorzugt in einer Menge, um eine lokale Konzentration von 60 µM bis etwa 70 µM bereitzustellen, mehr bevorzugt in einer Menge, um eine lokale Konzentration von 62 µM bis 66 µM Kaffeesäureethylester an der Stelle bereitzustellen, die diesen benötigt, wobei mehr bevorzugt die Zusammensetzung den Kaffeesäureethylester in einer Menge von 1 µg/g der Zusammensetzung bis 15 µg/g der Zusammensetzung, mehr bevorzugt in einer Menge von 2,5 µg/g der Zusammensetzung bis 10 µg/g der Zusammensetzung und noch mehr bevorzugt in einer Menge von 5 µg/g der Zusammensetzung bis 7,5 µg/g der Zusammensetzung umfasst.

9. Arzneimittelzusammensetzung nach einem der Ansprüche 3 bis 8, wobei die Zusammensetzung die E-Selektin-Transkription nach Aktivierung mit Interleukin 1β um mindestens 30 %, vorzugsweise um mindestens 40 %, mehr bevorzugt um mindestens 45 %, mehr bevorzugt um mindestens 48 %, noch mehr bevorzugt um mindestens 50 %, inhibiert.

10. Arzneimittelzusammensetzung nach einem der Ansprüche 3 bis 9, wobei die Zusammensetzung eine topische Zusammensetzung oder eine Zusammensetzung zur internen Verabreichung ist, wobei vorzugsweise die topische Zusammensetzung in Form eines Balsams, einer Salbe, einer Paste, einer viskosen Flüssigkeit, einer Lotion, eines Gels, einer Creme, einer Mousse, eines Schaums, einer Beschichtung oder eines anderen Klebemittels, aufgebracht auf einen Streifen oder einen auflösbaren Streifen oder eine Kombination davon vorliegt und/oder wobei vorzugsweise die Zusammensetzung zur internen Verabreichung in Form einer Kapsel, einer Tablette, einer Lutschtablette, einer Kautablette, eines Kaugummis, eines Sirups, einer Flüssigkeit, einer Mousse oder einer Kombination davon vorliegt.

11. Arzneimittelzusammensetzung nach Anspruch 10, wobei der pharmazeutisch verträgliche Träger eine Wasser-in-ÖI-Emulsion ist, die mindestens ein Trägergrundmaterial, ein Tensid und Wasser umfasst, wobei vorzugsweise das Trägergrundmaterial aus nichttoxischem Öl, nichttoxischen Speiseölen, gesättigten oder ungesättigten Fettalkoholen, aliphatischen Kohlenwasserstoffen, langkettigen Triglyceriden, Fettsäureestern und Mischungen davon ausgewählt ist, wobei mehr bevorzugt das Trägergrundmaterial Erdnussöl ist.

12. Arzneimittelzusammensetzung nach einem der Ansprüche 3 bis 11, wobei die Zusammensetzung ferner mindestens eines von Konservierungsmitteln, Farbstoffen, Duftstoffen, Stabilisierungsmitteln, Puffersubstanzen oder einer Kombination davon umfasst.

13. Arzneimittelzusammensetzung nach einem der Ansprüche 3 bis 12, wobei die Zusammensetzung mindestens das Arylnaphthalenlignan Comfreyn A nach Anspruch 3, Kaffeesäureethylester, Malaxinsäure und Globoidnan A in einem pharmazeutisch verträglichen Träger umfasst, der mindestens ein nichttoxisches Speiseöl, mindestens einen Emulgator, mindestens eine Puffersubstanz, Wasser und Konservierungsmittel umfasst, wobei vorzugsweise die Zusammensetzung Comfreyn A in einer Menge umfasst, um eine lokale Konzentration von etwa 50 µM Comfreyn A an der Stelle bereitzustellen, an der dieses benötigt wird, und Kaffeesäureethylester in einer Menge, um eine lokale Konzentration von etwa 65 µM Kaffeesäureethylester an der Stelle bereitzustellen, an der dieses benötigt wird, wobei mehr bevorzugt die Zusammensetzung Comfreyn A in einer Menge von 3 µg/g der Zusammensetzung bis 5 µg/g der Zusammensetzung und Kaffeesäureethylester in einer Menge von 5 µg/g der Zusammensetzung bis 7,5 µg/g der Zusammensetzung in einem pharmazeutischen Träger umfasst, der vorzugsweise mindestens Erdnussöl, mindestens einen Emulgator, mindestens eine Puffersubstanz, Wasser, Duftstoffe und Konservierungsmittel umfasst.

14. Ein oder mehrere Arylnaphthalenlignan(e) nach einem der Ansprüche 1 oder 2 und die Arzneimittelzusammensetzung nach einem der Ansprüche 3 bis 13 zur Verwendung bei der Hemmung und/oder Behandlung von Entzündungen, vorzugsweise zur Behandlung von gebrochenen Knochen, Sehnenschäden, schmerzhaften Gelenken und Muskeln, akuten Schmerzen des oberen und unteren Rückens, Gonarthrose, stumpfen Verletzungen, zur Verringerung der Schwellung von Gelenkdistorsionen, zur Verbesserung der Gelenkmobilität bei osteoarthrotischen Gelenken und/oder Kombinationen davon.

15. Verwendung des Arylnaphthalenlignans Comfreyn A nach Anspruch 2 als Markersubstanz für die quantitative Analyse des Wirkstoffs in Produkten, in denen Beinwellwurzel verwendet wird und/oder die aus Beinwellwurzel extrahiert sind.

## Revendications

1. Une ou plusieurs arylnaphtalène lignane(s) ayant la structure générale telle que montrée dans la Formule I 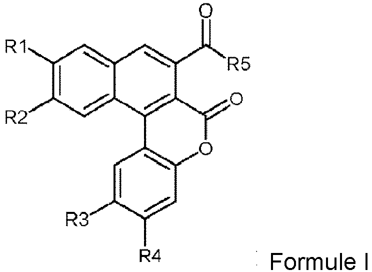 dans laquelle/lesquelles R1, R2, R3, et R4 sont indépendamment choisis parmi les hydrogène, hydroxy, méthyle, méthoxy, éthoxy, propoxy, butoxy ou pentoxy ; et
dans laquelle/lesquelles R5 est choisi parmi des résidus hydroxy, méthoxy, éthoxy, propoxy, butoxy, pentoxy, prénoxy, isoprénoxy, féruloyle, cafféoyle, ou coumaroyle.

2. Arylnaphtalène lignane(s) selon les revendication 1, dans laquelle/lesquelles R1, R2, R3 et R4 sont chacun hydroxy et R5 est éthoxy.

3. Composition pharmaceutique comprenant
a) un agent actif comprenant une ou plusieurs parmi la ou les arylnaphtalène lignane(s) selon l'une quelconque des revendications 1 ou 2 ; et
b) un support acceptable sur le plan pharmaceutique.

4. Composition pharmaceutique selon la revendication 3 comprenant en outre un agent actif supplémentaire, dans laquelle l'agent actif supplémentaire est de préférence un analgésique, un agent actif avec une activité anti-inflammatoire, un stimulateur d'activité ou une combinaison de ceux-ci, plus préférablement dans laquelle l'agent actif supplémentaire est un analgésique naturel, plus préférablement dans laquelle l'agent actif supplémentaire est un analgésique naturel extrait de *Symphytum officinale* L.

5. Composition pharmaceutique selon la revendication 4 dans laquelle l'agent actif supplémentaire comprend de l'ester éthylique d'acide caféique, de l'acide malaxinique, du globoidnan A, de l'aldéhyde protocatéchique, un extrait de racine de *Symphytum officinale* L. ou une combinaison de ceux-ci, de préférence dans laquelle l'agent actif supplémentaire comprend de l'ester éthylique d'acide caféique, de l'acide malaxinique, du globoidnan A ou une combinaison de ceux-ci.

6. Composition pharmaceutique selon les revendications 4 ou 5, dans laquelle l'agent actif supplémentaire comprend en outre de l'allantoïne, du glucoside d'acide p-hydroxybenzoïque, du 5-hydroxyméthyl-2-furfural, de l'acide protocatéchique, de l'acide p-hydroxybenzoïque, de l'acide caféique, du globoidnan B, du 3-carboxy-6, 7-dihydroxy-1-(3', 4'-dihydroxyphényl)-naphtalène, de la (+)-rabdosiine, de l'acide rosmarinique, de l'acide alpha-hydroxyhydrocaféique, de la ternifoliuslignane D, de l'acide linolénique, de l'acide linoléique, de l'acide hydroxypalmitique ou une combinaison de ceux-ci, comprend de préférence du globoidnan B, de la ternifoliuslignane D, du 3-carboxy-6, 7-dihydroxy-1-(3', 4'-dihydroxyphényl)-naphtalène, de la rabdosiine, et/ou une combinaison de ceux-ci.

7. Composition pharmaceutique selon l'une quelconque des revendications 3 à 6, dans laquelle la composition comprend l'agent actif à base d'une ou plusieurs arylnaphtalène lignane(s) en une quantité sûre et efficace sur le plan pharmaceutique, de préférence en une quantité permettant de fournir une concentration locale allant de 30 µM à 65 µM, plus préférablement en une quantité permettant de fournir une concentration locale allant de 40 µM à 60 µM, plus préférablement en une quantité permettant de fournir une concentration locale allant de 45 µM à environ 55 µM, plus préférablement en une quantité permettant de fournir une concentration locale allant de 48 µM à 52 µM d'agent actif à l'emplacement qui en a besoin, plus préférablement dans laquelle la composition comprend l'agent actif à base d'une ou plusieurs arylnaphtalène lignane(s) en une quantité allant de 1 µg/g de composition à 10 µg/g de composition, plus préférablement en une quantité allant de 2 µg/g de composition à 7, 5 µg/g de composition, et encore plus préférablement en une quantité allant de 3 µg/g de composition à 5 µg/g de composition.

8. Composition pharmaceutique selon l'une quelconque des revendications 5 à 7, dans laquelle la composition comprend de l'ester éthylique d'acide caféique en une quantité sûre et efficace, de préférence en une quantité permettant de fournir une concentration locale allant de 50 µM à 80 µM, plus préférablement en une quantité permettant de fournir une concentration locale allant de 55 µM à 75 µM, plus préférablement en une quantité permettant de fournir une concentration locale allant de 60 µM à environ 70 µM, plus préférablement en une quantité permettant de fournir une concentration locale allant de 62 µM à 66 µM d'ester éthylique d'acide caféique à l'emplacement qui en a besoin, plus préférablement dans laquelle la composition comprend l'ester éthylique d'acide caféique en une quantité allant de 1 µg/g de composition à 15 µg/g de composition, plus préférablement en une quantité allant de 2, 5 µg/g de composition à 10 µg/g de composition, et encore plus préférablement en une quantité allant de 5 µg/g de composition à 7, 5 µg/g de composition.

9. Composition pharmaceutique selon l'une quelconque des revendications 3 à 8, dans laquelle la composition inhibe une transcription de E-sélectine après activation avec de l'interleukine 1β d'au moins 30 %, de préférence d'au moins 40 %, plus préférablement d'au moins 45 %, plus préférablement d'au moins 48 %, encore plus préférablement d'au moins 50 %.

10. Composition pharmaceutique selon l'une quelconque des revendications 3 à 9, dans laquelle la composition est une composition topique ou une composition pour administration interne, de préférence dans laquelle la composition topique est sous la forme d'une pommade, d'un onguent, d'une pâte, d'un liquide visqueux, d'une lotion, d'un gel, d'une crème, d'une mousse, d'une écume, d'un revêtement ou de n'importe quel autre produit adhésif, appliqué à une bandelette ou une bandelette soluble ou une combinaison de ceux-ci et/ou dans laquelle la composition pour administration interne est de préférence sous forme d'une gélule, d'un comprimé, d'une dragée, d'un comprimé à mâcher, d'un chewing-gum, d'un sirop, d'un liquide, d'une mousse ou d'une combinaison de ceux-ci.

11. Composition pharmaceutique selon la revendication 10, dans laquelle le support acceptable sur le plan pharmaceutique est une émulsion eau-dans-huile comprenant au moins un matériau de base de support, un agent tensioactif et de l'eau, de préférence dans laquelle le matériau de base de support est choisi parmi une huile non toxique, des huiles comestibles non toxiques, des alcools gras saturés ou insaturés, des hydrocarbures aliphatiques, des triglycérides à longue chaîne, des esters gras, et des mélanges de ceux-ci, plus préférablement dans laquelle le matériau de base de support est de l'huile d'arachide.

12. Composition pharmaceutique selon l'une quelconque des revendications 3 à 11, dans laquelle la composition comprend en outre au moins un parmi des conservateurs, des teintures, des parfums, des agents stabilisants, des agents tampons ou une combinaison de ceux-ci.

13. Composition pharmaceutique selon l'une quelconque des revendications 3 à 12, dans laquelle la composition comprend au moins l'arylnaphtalène lignane consoude A selon la revendication 3, de l'ester éthylique d'acide caféique, de l'acide malaxinique et du globoidnan A dans un support acceptable sur le plan pharmaceutique comprenant au moins une huile comestible non toxique, au moins un émulsifiant, au moins un agent tampon, de l'eau et des conservateurs, de préférence dans laquelle la composition comprend de la consoude A en une quantité permettant de fournir une concentration locale d'environ 50 µM de consoude A à l'emplacement qui en a besoin et de l'ester éthylique d'acide caféique en une quantité permettant de fournir une concentration locale d'environ 65 µM d'ester éthylique d'acide caféique à l'emplacement qui en a besoin, plus préférablement dans laquelle la composition comprend de la consoude A en une quantité allant de 3 µg/g de composition à 5 µg/g de composition et de l'ester éthylique d'acide caféique en une quantité allant de 5 µg/g de composition à 7, 5 µg/g de composition, dans un support pharmaceutique comprenant de préférence au moins de l'huile d'arachide, au moins un émulsifiant, au moins un agent tampon, de l'eau, des parfums et des conservateurs.

14. Une ou plusieurs arylnaphtalène lignane(s) selon l'une quelconque des revendications 1 ou 2 et composition pharmaceutique selon l'une quelconque des revendications 3 à 13 pour utilisation dans l'inhibition et/ou le traitement d'une inflammation, de préférence pour le traitement d'os fracturés, de dommages aux tendons, de douleurs articulaires et musculaires, de douleurs dorsales aiguës supérieures et inférieures, de gonarthrose, de blessures contondantes, pour la réduction du gonflement de distorsions articulaires, pour l'amélioration de mobilité articulaire dans des articulations arthrosiques et/ou des combinaisons de ceux-ci.

15. Utilisation de l'arylnaphtalène lignane consoude A selon la revendication 2 en tant que substance de marquage pour une analyse quantitative de la substance active dans des produits utilisant et/ou extraits de racine de consoude.


### SEQUENCE LISTING

<110> The Procter & Gamble Company
<120> Comfreyn, arylnaphthalene lignans that inhibit pro-inflammatory gene expression, and pharmaceutic compositions comprising them
<130> CM05167FQ
<160> 4
<170> PatentIn version 3.5
<210> 1
   <211> 19
   <212> DNA
   <213> glyceraldehyde 3-phosphate dehydrogenase (GAPDH)
<400> 1
   agaaggctgg ggctcattt 19
<210> 2
   <211> 20
   <212> DNA
   <213> glyceraldehyde 3-phosphate dehydrogenase (GAPDH)
<400> 2
   ctaagcagtt ggtggtgcag 20
<210> 3
   <211> 19
   <212> DNA
   <213> E-Selectin (SELE)
<400> 3
   cctgtgaagc tcccactga 19
<210> 4
   <211> 21
   <212> DNA
   <213> E-Selectin (SELE)
<400> 4
   ggcttttggt agcttccatc t 21